# EUROPEAN PATENT APPLICATION

(11) **EP 2 644 126 A2**
(43) Date of publication of application: **02.10.2013**
(21) Application number: 13161496.8
(22) Date of filing: 27.03.2013
(51) Int. Cl.: A61B 17/072

(54) **Devices and methods for attaching tissue thickness compensating materials to surgical stapling instruments**

(30) Priority: 28.03.2012 US 201213433132
(71) Applicant: Ethicon Endo-Surgery, Inc., Cincinnati, OH 45242 (US)
(72) Inventor: Schmid, Katherine J., Cincinnati, Ohio 45241 (US); Aronhalt, Taylor W., Loveland, Ohio 45140 (US); Morgan, Jerome R., Cincinnati, Ohio 45236 (US); Shelton, IV Frederick E., Hillsboro, Ohio 45133 (US); Scheib, Charles J., Loveland, Ohio 45140 (US); Stammen, John L., Cincinnati, Ohio 45241 (US); Ortiz, Mark S., Milford, Ohio 45150 (US); Aldridge, Jeffrey L., Lebanon, Ohio 45036 (US)
(74) Representative: Carpmaels & Ransford LLP

(57) **Abstract**

Surgical stapling instruments are disclosed. At least one embodiment includes a first jaw that supports a plurality of surgical staples and a second jaw that is movable relative to the first jaw. Various arrangements include a tissue thickness compensator that is configured to be captured within the surgical staples and assume different compressed heights within different surgical staples upon application of a firing motion to the surgical staples. Various attachment protrusion arrangements are disclosed to mechanically removably attach the tissue thickness compensator to the first or second jaw.

## Description

### BACKGROUND

The present invention relates to surgical instruments and, in various embodiments, to surgical cutting and stapling instruments and staple cartridges therefor that are designed to cut and staple tissue.

### SUMMARY

The following is a non-exhaustive list of embodiments of the present invention that are or may be claimed.
1. A surgical stapling instrument comprising:
   a first jaw supporting a plurality of surgical staples therein operably responsive to an application of a firing motion thereto;
   a second jaw movably supported relative to the first jaw such that a portion of the second jaw is movable into confronting relationship relative to the first jaw upon application of a closing motion to the second jaw;
   a tissue thickness compensator configured to be captured within the surgical staples and assume different compressed heights within different surgical staples upon application of the firing motion to the surgical staples; and
   at least one attachment protrusion on one of the first and second jaws for removably mechanically affixing a corresponding portion of the tissue thickness compensator thereto.
2. The surgical stapling instrument of embodiment 1 wherein the tissue thickness compensator comprises a fibrous body structure and wherein the at least one attachment protrusion comprises a plurality of attachment protrusions configured to removably retainingly engage the fibrous body structure.
3. The surgical stapling instrument of embodiment 1 or embodiment 2 wherein the tissue thickness compensator comprises a compressible body encapsulated by a film and wherein the at least one attachment protrusion is configured to removably retainingly engage at least one other corresponding other attachment protrusion on the film.
4. The surgical stapling instrument of any preceding embodiment wherein the at least one attachment protrusion comprises at least one area of:
   attachment protrusions having a pyramidal shape;
   attachment protrusions having a hexagonal shape;
   attachment protrusions including a protruding body portion and a distal end portion that is not coaxial with the body portion;
   attachment columns including a height that is greater than its cross-sectional area;
   hook-shaped attachment protrusions;
   loop-shaped protrusions;
   T-shaped protrusions; and
   microfibers.
5. The surgical stapling instrument of embodiment 3 wherein the film includes at least one area of fluid-wicking members formed thereon.
6. The surgical stapling instrument of any preceding embodiment wherein the tissue thickness compensator comprises a compressible body fabricated from woven material.
7. The surgical instrument of any one of claims 1-5 wherein the tissue thickness compensator comprises a compressible body fabricated from non-woven material.
8. The surgical stapling instrument of any preceding embodiment wherein the first jaw comprises a cartridge body defining a deck surface including a plurality of staple cavities through the deck surface and wherein the plurality of surgical staples are positioned within the staple cavities.
9. The surgical stapling instrument of embodiment 8 wherein the at least one attachment protrusion protrudes from the deck surface.
10. The surgical stapling instrument of embodiment 8 or embodiment 9 wherein the at least one attachment protrusion is integrally formed on the deck surface.
11. The surgical stapling instrument of any one of claims 8-10 wherein the tissue thickness compensator comprises a fibrous body structure and wherein the at least one attachment protrusion comprises a plurality of attachment protrusions configured to removably retainingly engage the fibrous body structure.
12. The surgical stapling instrument of any one of claims 8-11 wherein said tissue thickness compensator comprises a compressible body encapsulated by a film and wherein the at least one attachment protrusion is configured to removably retainingly engage corresponding at least one other attachment protrusion on the film.
13. The surgical stapling instrument of any one of claims 8-12 wherein the cartridge body includes a longitudinally extending deck slot formed through the deck surface for receiving a portion of a tissue cutting member therein and wherein the at least one attachment protrusion comprises at least one area of attachment protrusions on one side of the deck slot and at least one other area of attachment protrusions on another side of the deck slot.
14. The surgical stapling instrument of any preceding embodiment wherein the second jaw comprises an anvil including a staple-forming surface thereon and wherein the at least one attachment protrusion protrudes from the staple forming surface.
15. The surgical stapling instrument of embodiment 14 wherein the at least one attachment protrusion comprises an area of attachment protrusions formed on a corresponding attachment carrier that is configured to be retainingly coupled to the anvil.
16. The surgical stapling instrument of embodiment 15 wherein each of the corresponding attachment carriers is configured to snappingly engage the anvil.
17. The surgical stapling instrument of embodiment 15 or embodiment 16 wherein the area of attachment protrusions are attached to the corresponding attachment carrier by adhesive.
18. The surgical stapling instrument of embodiment 15 or embodiment 16 wherein the area of attachment protrusions are integrally formed on the corresponding attachment carrier.
19. A surgical stapling instrument, comprising:
   a staple cartridge, comprising:
      a cartridge body defining a deck surface having a plurality of staple cavities through the deck surface;
      a plurality of staples positioned within the staple cavities;
   an anvil including a staple-forming surface thereon and being movably supported relative to the staple cartridge to bring the staple forming surface in confronting relationship relative to the deck surface of the cartridge body in response to closing motions applied thereto;
   a tissue thickness compensator configured to be captured within the staples and assume different compressed heights within different staples; and
   at least one area of attachment protrusions on the staple forming surface of the anvil for removably attaching the tissue thickness compensator thereto.
20. A surgical stapling instrument, comprising:
   a staple cartridge, comprising:
      a cartridge body defining a deck surface including a plurality of staple cavities through the deck surface;
      a plurality of staples positioned within the staple cavities;
   an anvil including a staple-forming surface thereon and being movably supported relative to the staple cartridge to bring the staple forming surface in confronting relationship relative to the deck surface of the cartridge body in response to closing motions applied thereto;
   a tissue thickness compensator configured to be captured within the staples and assume different compressed heights within different staples; and
   at least one area of attachment protrusions on the deck surface for removably attaching the tissue thickness compensator thereto.
21. A surgical stapling instrument comprising:
   a first jaw supporting a plurality of surgical staples therein operably responsive to an application of a firing motion thereto;
   a second jaw movably supported relative to the first jaw such that a portion of the second jaw is movable into confronting relationship relative to the first jaw upon application of a closing motion to the second jaw;
   a tissue thickness compensator configured to be captured within the surgical staples and assume different compressed heights within different surgical staples upon application of the firing motion to the surgical staples, the tissue thickness compensator configured to establish a suction retention force between the tissue thickness compensator and one of the first and second jaws.
22. A surgical staple cartridge, comprising:
   a cartridge body defining a deck surface including a plurality of staple cavities through the deck surface;
   a plurality of staples positioned within the staple cavities; and
   at least one area of attachment protrusions on the deck surface for removably attaching a tissue thickness compensator thereto.

### SUMMARY

In accordance with at least one general form, there is provided a surgical stapling instrument. In at least one form, the surgical stapling instrument includes a first jaw that supports a plurality of surgical staples that are operably responsive to an application of a firing motion thereto. A second jaw is movably supported relative to the first jaw such that a portion of the second jaw is movable into confronting relationship relative to the first jaw upon application of a closing motion to the second jaw. A tissue thickness compensator is configured to be captured within the surgical staples and assume different compressed heights within different surgical staples upon application of the firing motion to the surgical staples. At least one attachment protrusion is provided on one of the first and second jaws for removably mechanically affixing the tissue thickness compensator thereto.

In accordance with at least one other general form, there is provided a surgical stapling instrument. In at least one form, the surgical stapling instrument includes a staple cartridge that comprises a cartridge body that defines a deck surface that has a plurality of staple extending therethrough. A plurality of staples are positioned within the staple cavities. The instrument further comprises an anvil that has a staple-forming surface and which is movably supported relative to the staple cartridge to bring the staple forming surface in confronting relationship relative to the deck surface of the cartridge body in response to closing motions applied thereto. A tissue thickness compensator is configured to be captured within the staples and assume different compressed heights within different staples. At least one area of attachment protrusions are provided on the staple forming surface of the anvil for removably attaching the tissue thickness compensator thereto.

In accordance with still another general form, there is provided a surgical stapling instrument. In at least one form, the surgical stapling instrument comprises a staple cartridge that comprises a cartridge body that defines a deck surface that has a plurality of staple extending therethrough. A plurality of staples are positioned within the staple cavities. The surgical stapling device further comprises an anvil that has having a staple-forming surface thereon and which is movably supported relative to the staple cartridge to bring the staple forming surface in confronting relationship relative to the deck surface of the cartridge body in response to closing motions applied thereto. A tissue thickness compensator is configured to be captured within the staples and assume different compressed heights within different staples. The device further includes at least one area of attachment protrusions on the deck surface for removably attaching the tissue thickness compensator thereto.

In accordance with still other general aspects, there is provided a surgical stapling instrument that has a first jaw that supporting a plurality of surgical staples therein that are operably responsive to an application of a firing motion thereto. The surgical stapling instrument further comprises a second jaw that is movably supported relative to the first jaw such that a portion of the second jaw is movable into confronting relationship relative to the first jaw upon application of a closing motion to the second jaw. The instrument further comprises a tissue thickness compensator that is configured to be captured within the surgical staples and assume different compressed heights within different surgical staples upon application of the firing motion to the surgical staples. In at least one embodiment, the tissue thickness compensator is further configured to establish a suction retention force between the tissue thickness compensator and one of the first and second jaws.

### BRIEF DESCRIPTION OF THE DRAWINGS

The features and advantages of this invention, and the manner of attaining them, will become more apparent and the invention itself will be better understood by reference to the following description of embodiments of the invention taken in conjunction with the accompanying drawings, wherein:

FIG. 1 is a side view of a surgical stapling instrument which may be used in connection with various embodiments;

FIG. 2 is a cross-sectional view of the end effector portion of the surgical stapling instrument of Figure 1 taken along line 2-2 in FIG.1;

FIG. 3 is an end perspective view of the end effector depicted in FIG.2 with the anvil thereof in an open position;

FIG. 4 is an exploded assembly view of a portion of the surgical stapling instrument of FIG. 1;

FIG. 5 is perspective view of a portion of an anvil of an embodiment;

FIG. 6 is a diagram illustrating a tissue thickness compensator which is compensating for different tissue thickness captured within different staples;

FIG. 7 is a diagram illustrating a tissue thickness compensator applying a compressive pressure to one or more vessels that have been transected by a staple line;

FIG. 8 is a diagram illustrating a circumstance wherein one or more staples have been improperly formed;

FIG. 9 is a diagram illustrating a tissue thickness compensator which could compensate for improperly formed staples;

FIG. 10 is a diagram illustrating a tissue thickness compensator positioned in a region of tissue in which multiple staples lines have intersected;

FIG. 11 is a diagram illustrating tissue captured within a staple;

FIG. 12 is a diagram illustrating tissue and a tissue thickness compensator captured within a staple;

FIG. 13 is a diagram illustrating tissue captured within a staple;

FIG. 14 is a diagram illustrating thick tissue and a tissue thickness compensator captured within a staple;

FIG. 15 is a diagram illustrating thin tissue and a tissue thickness compensator captured within a staple;

FIG. 16 is a diagram illustrating tissue having an intermediate thickness and a tissue thickness compensator captured within a staple;

FIG. 17 is a diagram illustrating tissue having another intermediate thickness and a tissue thickness compensator captured within a staple;

FIG. 18 is a diagram illustrating thick tissue and a tissue thickness compensator captured within a staple;

FIG. 19 is a bottom view of the anvil of FIG. 5 and a tissue thickness compensator embodiment;

FIG. 20 is a perspective view of a portion of an area of protrusions of an embodiment;

FIG. 21 is a perspective view of a portion of an area of protrusions of another embodiment;

FIG. 22 is a perspective view of a portion of an area of protrusions of another embodiment;

FIG. 23 is a partial perspective view of an anvil and tissue thickness compensator embodiment;

FIG. 24 is a partial exploded assembly view of an anvil and tissue thickness compensator embodiment;

FIG. 25 is a cross-sectional view of a portion of the anvil of FIG. 11 with a tissue thickness compensator attached thereto;

FIG. 26 is a partial exploded assembly view of another anvil and tissue thickness compensator embodiment;

FIG. 27 is a cross-sectional view of a portion of the anvil of FIG. 26 with a tissue thickness compensator attached thereto;

FIG. 28 is a partial exploded assembly view of another anvil and tissue thickness compensator embodiment;

FIG. 29 is a cross-sectional view of a portion of the anvil of FIG. 28 with a tissue thickness compensator attached thereto;

FIG. 30 is a partial exploded assembly view of another anvil and tissue thickness compensator embodiment;

FIG. 31 is a perspective view of a portion of the anvil of FIG. 30;

FIG. 32 is a partial exploded assembly view of another anvil and tissue thickness compensator embodiment;

FIG. 33 is a perspective view of a portion of the anvil of FIG. 32;

FIG. 34 is a partial exploded assembly view of another anvil and tissue thickness compensator embodiment;

FIG. 35 is a perspective view of a portion of the anvil of FIG. 34;

FIG. 36 is a partial side view of another end effector embodiment and tissue thickness compensator embodiment;

FIG. 37 is an enlarged view of a portion of the end effector and tissue thickness compensator of FIG. 36;

FIG. 38 is a partial exploded assembly view of another anvil and tissue thickness compensator embodiment;

FIG. 39 is a cross-sectional view of a mold embodiment;

FIG. 40 is a perspective view of a portion of the tissue thickness compensator of FIG. 38; and

FIG. 41 is a perspective view of another end effector embodiment and tissue thickness compensator embodiment.

Corresponding reference characters indicate corresponding parts throughout the several views. The exemplifications set out herein illustrate certain embodiments of the invention, in one form, and such exemplifications are not to be construed as limiting the scope of the invention in any manner.

### DETAILED DESCRIPTION

The Applicant of the present application also owns the U.S. Patent Applications identified below which are each herein incorporated by reference in their respective entirety:
U.S. Patent Application Serial No. 12/894,311, entitled SURGICAL INSTRUMENTS WITH RECONFIGURABLE SHAFT SEGMENTS (Attorney Docket No. END6734USNP/100058);
U.S. Patent Application Serial No. 12/894,340, entitled SURGICAL STAPLE CARTRIDGES SUPPORTING NON-LINEARLY ARRANGED STAPLES AND SURGICAL STAPLING INSTRUMENTS WITH COMMON STAPLE-FORMING POCKETS (Attorney Docket No. END6735USNP/100059);
U.S. Patent Application Serial No. 12/894,327, entitled JAW CLOSURE ARRANGEMENTS FOR SURGICAL INSTRUMENTS (Attorney Docket No. END6736USNP/100060);
U.S. Patent Application Serial No. 12/894,351, entitled SURGICAL CUTTING AND FASTENING INSTRUMENTS WITH SEPARATE AND DISTINCT FASTENER DEPLOYMENT AND TISSUE CUTTING SYSTEMS (Attorney Docket No. END6839USNP/100524);
U.S. Patent Application Serial No. 12/894,338, entitled IMPLANTABLE FASTENER CARTRIDGE HAVING A NON-UNIFORM ARRANGEMENT (Attorney Docket No. END6840USNP/100525);
U.S. Patent Application Serial No. 12/894,369, entitled IMPLANTABLE FASTENER CARTRIDGE COMPRISING A SUPPORT RETAINER (Attorney Docket No. END6841USNP/100526);
U.S. Patent Application Serial No. 12/894,312, entitled IMPLANTABLE FASTENER CARTRIDGE COMPRISING MULTIPLE LAYERS (Attorney Docket No. END6842USNP/100527);
U.S. Patent Application Serial No. 12/894,377, entitled SELECTIVELY ORIENTABLE IMPLANTABLE FASTENER CARTRIDGE (Attorney Docket No. END6843USNP/100528);
U.S. Patent Application Serial No. 12/894,339, entitled SURGICAL STAPLING INSTRUMENT WITH COMPACT ARTICULATION CONTROL ARRANGEMENT (Attorney Docket No. END6847USNP/100532);
U.S. Patent Application Serial No. 12/894,360, entitled SURGICAL STAPLING INSTRUMENT WITH A VARIABLE STAPLE FORMING SYSTEM (Attorney Docket No. END6848USNP/100533);
U.S. Patent Application Serial No. 12/894,322, entitled SURGICAL STAPLING INSTRUMENT WITH INTERCHANGEABLE STAPLE CARTRIDGE ARRANGEMENTS (Attorney Docket No. END6849USNP/100534);
U.S. Patent Application Serial No. 12/894,350, entitled SURGICAL STAPLE CARTRIDGES WITH DETACHABLE SUPPORT STRUCTURES AND SURGICAL STAPLING INSTRUMENTS WITH SYSTEMS FOR PREVENTING ACTUATION MOTIONS WHEN A CARTRIDGE IS NOT PRESENT (Attorney Docket No. END6855USNP/100540);
U.S. Patent Application Serial No. 12/894,383, entitled IMPLANTABLE FASTENER CARTRIDGE COMPRISING BIOABSORBABLE LAYERS (Attorney Docket No. END6856USNP/100541);
U.S. Patent Application Serial No. 12/894,389, entitled COMPRESSIBLE FASTENER CARTRIDGE (Attorney Docket No. END6857USNP/100542);
U.S. Patent Application Serial No. 12/894,345, entitled FASTENERS SUPPORTED BY A FASTENER CARTRIDGE SUPPORT (Attorney Docket No. END6858USNP/100543);
U.S. Patent Application Serial No. 12/894,306, entitled COLLAPSIBLE FASTENER CARTRIDGE (Attorney Docket No. END6859USNP/100544);
U.S. Patent Application Serial No. 12/894,318, entitled FASTENER SYSTEM COMPRISING A PLURALITY OF CONNECTED RETENTION MATRIX ELEMENTS (Attorney Docket No. END6860USNP/100546);
U.S. Patent Application Serial No. 12/894,330, entitled FASTENER SYSTEM COMPRISING A RETENTION MATRIX AND AN ALIGNMENT MATRIX (Attorney Docket No. END6861USNP/100547);
U.S. Patent Application Serial No. 12/894,361, entitled FASTENER SYSTEM COMPRISING A RETENTION MATRIX (Attorney Docket No. END6862USNP/100548);
U.S. Patent Application Serial No. 12/894,367, entitled FASTENING INSTRUMENT FOR DEPLOYING A FASTENER SYSTEM COMPRISING A RETENTION MATRIX (Attorney Docket No. END6863USNP/100549);
U.S. Patent Application Serial No. 12/894,388, entitled FASTENER SYSTEM COMPRISING A RETENTION MATRIX AND A COVER (Attorney Docket No. END6864USNP/100550);
U.S. Patent Application Serial No. 12/894,376, entitled FASTENER SYSTEM COMPRISING A PLURALITY OF FASTENER CARTRIDGES (Attorney Docket No. END6865USNP/100551);
U.S. Patent Application Serial No. 13/097,865, entitled SURGICAL STAPLER ANVIL COMPRISING A PLURALITY OF FORMING POCKETS (Attorney Docket No. END6735USCIP1/100059CIP1);
U.S. Patent Application Serial No. 13/097,936, entitled TISSUE THICKNESS COMPENSATOR FOR A SURGICAL STAPLER (Attorney Docket No. END6736USCIP1/100060CIP1);
U.S. Patent Application Serial No. 13/097,954, entitled STAPLE CARTRIDGE COMPRISING A VARIABLE THICKNESS COMPRESSIBLE PORTION (Attorney Docket No. END6840USCIP1/100525CIP1);
U.S. Patent Application Serial No. 13/097,856, entitled STAPLE CARTRIDGE COMPRISING STAPLES POSITIONED WITHIN A COMPRESSIBLE PORTION THEREOF (Attorney Docket No. END6841USCIP1/100526CIP1);
U.S. Patent Application Serial No. 13/097,928, entitled TISSUE THICKNESS COMPENSATOR COMPRISING DETACHABLE PORTIONS (Attorney Docket No. END6842USCIP1/100527CIP1);
U.S. Patent Application Serial No. 13/097,891, entitled TISSUE THICKNESS COMPENSATOR FOR A SURGICAL STAPLER COMPRISING AN ADJUSTABLE ANVIL (Attorney Docket No. END6843USCIP1/100528CIP1);
U.S. Patent Application Serial No. 13/097,948, entitled STAPLE CARTRIDGE COMPRISING AN ADJUSTABLE DISTAL PORTION (Attorney Docket No. END6847USCIP1/100532CIP1);
U.S. Patent Application Serial No. 13/097,907, entitled COMPRESSIBLE STAPLE CARTRIDGE ASSEMBLY (Attorney Docket No. END6848USCIP1/100533CIP1);
U.S. Patent Application Serial No. 13/097,861, entitled TISSUE THICKNESS COMPENSATOR COMPRISING PORTIONS HAVING DIFFERENT PROPERTIES (Attorney Docket No. END6849USCIP1/100534CIP1);
U.S. Patent Application Serial No. 13/097,869, entitled STAPLE CARTRIDGE LOADING ASSEMBLY (Attorney Docket No. END6855USCIP1/100540CIP1);
U.S. Patent Application Serial No. 13/097,917, entitled COMPRESSIBLE STAPLE CARTRIDGE COMPRISING ALIGNMENT MEMBERS (Attorney Docket No. END6856USCIP1/100541CIP1);
U.S. Patent Application Serial No. 13/097,873, entitled STAPLE CARTRIDGE COMPRISING A RELEASABLE PORTION (Attorney Docket No. END6857USCIP1/100542CIP1);
U.S. Patent Application Serial No. 13/097,938, entitled STAPLE CARTRIDGE COMPRISING COMPRESSIBLE DISTORTION RESISTANT COMPONENTS (Attorney Docket No. END6858USCIP1/100543CIP1);
U.S. Patent Application Serial No. 13/097,924, entitled STAPLE CARTRIDGE COMPRISING A TISSUE THICKNESS COMPENSATOR (Attorney Docket No. END6859USCIP1/100544CIP1);
U.S. Patent Application Serial No. 13/242,029, entitled SURGICAL STAPLER WITH FLOATING ANVIL (Attorney Docket No. END6841USCIP2/100526CIP2);
U.S. Patent Application Serial No. 13/242,066, entitled CURVED END EFFECTOR FOR A STAPLING INSTRUMENT (Attorney Docket No. END6841USCIP3/100526CIP3);
U.S. Patent Application Serial No. 13/242,086, entitled STAPLE CARTRIDGE INCLUDING COLLAPSIBLE DECK (Attorney Docket No. END7020USNP/110374);
U.S. Patent Application Serial No. 13/241,912, entitled STAPLE CARTRIDGE INCLUDING COLLAPSIBLE DECK ARRANGEMENT (Attorney Docket No. END7019USNP/110375);
U.S. Patent Application Serial No. 13/241,922, entitled SURGICAL STAPLER WITH STATIONARY STAPLE DRIVERS (Attorney Docket No. END7013USNP/110377);
U.S. Patent Application Serial No. 13/241,637, entitled SURGICAL INSTRUMENT WITH TRIGGER ASSEMBLY FOR GENERATING MULTIPLE ACTUATION MOTIONS (Attorney Docket No. END6888USNP3/110378); and
U.S. Patent Application Serial No. 13/241,629, entitled SURGICAL INSTRUMENT WITH SELECTIVELY ARTICULATABLE END EFFECTOR (Attorney Docket No. END6888USNP2/110379).

The Applicant of the present application also owns the U.S. Patent Applications identified below which were filed on even date herewith and which are each herein incorporated by reference in their respective entirety:
U.S. Application Serial No. _, entitled TISSUE THICKNESS COMPENSATOR COMPRISING A PLURALITY OF CAPSULES, (Attorney Docket No. END6864USCIP1/100550CIP1);
U.S. Application Serial No. _, entitled TISSUE THICKNESS COMPENSATOR COMPRISING A PLURALITY OF LAYERS, (Attorney Docket No. END6864USCIP2/100550CIP2);
U.S. Application Serial No. _, entitled EXPANDABLE TISSUE THICKNESS COMPENSATOR, (Attorney Docket No. END6843USCIP2/100528CIP2).
U.S. Application Serial No. _, entitled TISSUE THICKNESS COMPENSATOR COMPRISING A RESERVOIR, (Attorney Docket No. END6843USCIP3/100528CIP3);
U.S. Application Serial No. _, entitled RETAINER ASSEMBLY INCLUDING A TISSUE THICKNESS COMPENSATOR, (Attorney Docket No. END6843USCIP4/100528CIP4);
U.S. Application Serial No. _, entitled TISSUE THICKNESS COMPENSATOR COMPRISING AT LEAST ONE MEDICAMENT, (Attorney Docket No. END6843USCIPS/100528CIP5);
U.S. Application Serial No. _, entitled TISSUE THICKNESS COMPENSATOR COMPRISING CONTROLLED RELEASE AND EXPANSION, (Attorney Docket No. END6843USCIP6/100528CIP6);
U.S. Application Serial No. _, entitled TISSUE THICKNESS COMPENSATOR COMPRISING FIBERS TO PRODUCE A RESILIENT LOAD, (Attorney Docket No. END6843USCIP7/100528CIP7);
U.S. Application Serial No. _, entitled TISSUE THICKNESS COMPENSATOR COMPRISING STRUCTURE TO PRODUCE A RESILIENT LOAD, (Attorney Docket No. END6843USCIPB/100528CIP8);
U.S. Application Serial No. _, entitled TISSUE THICKNESS COMPENSATOR COMPRISING RESILIENT MEMBERS, (Attorney Docket No. END6843USCIP9/100528CIP9);
U.S. Application Serial No. _, entitled METHODS FOR FORMING TISSUE THICKNESS COMPENSATOR ARRANGEMENTS FOR SURGICAL STAPLERS, (Attorney Docket No. END6843USCIP10/100528CP10);
U.S. Application Serial No. _, entitled TISSUE THICKNESS COMPENSATORS, (Attorney Docket No. END6843USCIP11/100528CP11);
U.S. Application Serial No. _, entitled LAYERED TISSUE THICKNESS COMPENSATOR, (Attorney Docket No. END6843USCIP12/100528CP12);
U.S. Application Serial No. _, entitled TISSUE THICKNESS COMPENSATORS FOR CIRCULAR SURGICAL STAPLERS, (Attorney Docket No. END6843USCIP13/100528CP13);
U.S. Application Serial No. _, entitled TISSUE THICKNESS COMPENSATOR COMPRISING CAPSULES DEFINING A LOW PRESSURE ENVIRONMENT, (Attorney Docket No. END7100USNP/110601);
U.S. Application Serial No. _, entitled TISSUE THICKNESS COMPENSATOR COMPRISED OF A PLURALITY OF MATERIALS, (Attorney Docket No. END7101USNP/110602);
U.S. Application Serial No. _, entitled MOVABLE MEMBER FOR USE WITH A TISSUE THICKNESS COMPENSATOR, (Attorney Docket No. END7107USNP/110603);
U.S. Application Serial No. _, entitled TISSUE THICKNESS COMPENSATOR COMPRISING A PLURALITY OF MEDICAMENTS, (Attorney Docket No. END7102USNP/110604);
U.S. Application Serial No. _, entitled TISSUE THICKNESS COMPENSATOR AND METHOD FOR MAKING THE SAME, (Attorney Docket No. END7103USNP/110605);
U.S. Application Serial No. _, entitled TISSUE THICKNESS COMPENSATOR COMPRISING CHANNELS, (Attorney Docket No. END7104USNP/110606);
U.S. Application Serial No. _, entitled TISSUE THICKNESS COMPENSATOR COMPRISING TISSUE INGROWTH FEATURES, (Attorney Docket No. END7105USNP/110607); and
U.S. Application Serial No. _, entitled DEVICES AND METHODS FOR ATTACHING TISSUE THICKNESS COMPENSATING MATERIALS TO SURGICAL STAPLING INSTRUMENTS, (Attorney Docket No. END7106USNP/110608).

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are nonlimiting exemplary embodiments. The features illustrated or described in connection with one exemplary embodiment may be combined with the features of other embodiments. Such modifications and variations are included within the scope of the present invention.

Any of the methods disclosed or claimed herein for manufacturing, forming or otherwise producing an article or product, may be employed to manufacture, form or otherwise produce all or part of the article or product in question, and where such a method is employed to manufacture, form or otherwise produce part of the article or product in question, the remainder of the article or product may be produced in any way, including by employing any of the other methods disclosed and claimed herein for manufacturing, forming or otherwise producing the article or product, and the various parts so produced may be combined in any manner. Similarly, any article or product disclosed or claimed herein may exist alone, or in combination with, or as an integral part of any other article or product so disclosed with which it is compatible. Thus, the particular features, structures, or characteristics illustrated or described in connection with one article, product or method may be combined, in whole or in part, with the features structures, or characteristics of one or more other compatible articles, products or methods without limitation. Such modifications and variations are included within the scope of the present invention.

Where it is disclosed herein, either with reference to a particular figure or otherwise, that a certain embodiment of the invention or a certain article, product or method may comprise certain structures, characteristics or features, it will be understood by the reader that this signifies that those structures, characteristics or features may be embodied in the article, product or method in question in any compatible combination. In particular, such a disclosure of a number of optional structures, characteristics or features shall be understood also to disclose all of those structures, characteristics or features in combination, except in the case of structures, characteristics or features that are disclosed as alternatives to one another. Where such structures, characteristics or features are disclosed as alternatives to one another, this shall be understood to disclose those alternatives as being substitutions for each other.

The terms "proximal" and "distal" are used herein with reference to a clinician manipulating the handle portion of the surgical instrument. The term "proximal" referring to the portion closest to the clinician and the term "distal" referring to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications, including in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongated shaft of a surgical instrument can be advanced.

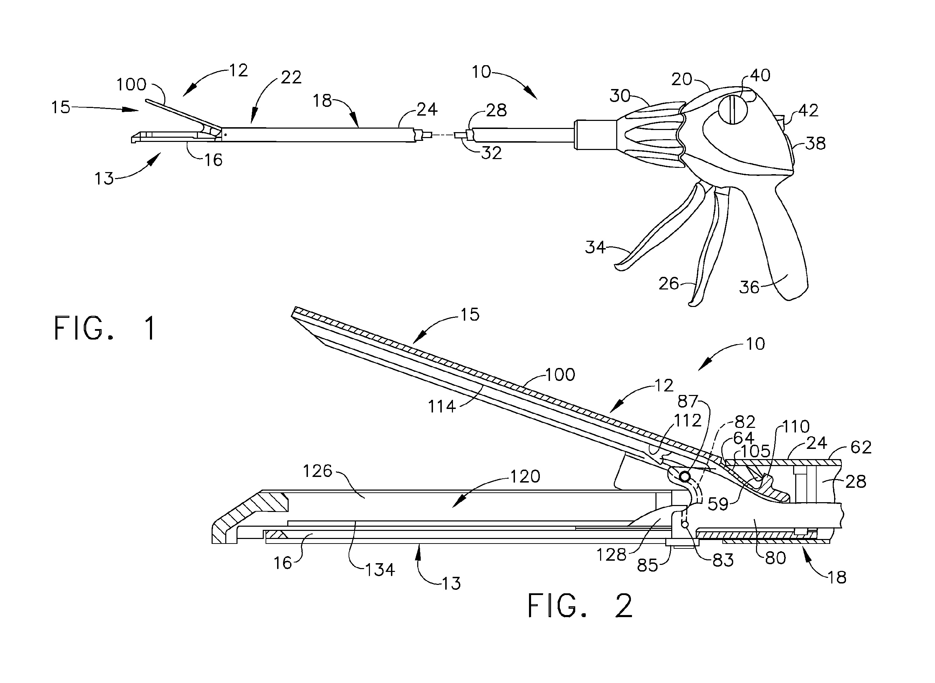

Turning to the Drawings wherein like numerals denote like components throughout the several views, FIGS. 1 and 2 depict a surgical stapling and severing instrument 10. An illustrative surgical stapling and severing instrument is described in greater detail in U.S. Patent No. 7,364,061, entitled "Surgical Stapling Instrument Incorporating a Multistroke Firing Position Indicator and Retraction Mechanism", issued April 29, 2008, the entire disclosure of which is herein incorporated by reference. A variety of other surgical stapling and severing instruments are known. As the present Detailed Description proceeds, those of ordinary skill in the art will understand that the unique and novel attributes of the present invention may be effectively employed in connection with various other forms of surgical stapling and severing instruments without departing from the spirit and scope of the present invention. For example, other forms of surgical stapling and severing instruments are disclosed in U.S. Patent No. 7,000,818, entitled "Surgical Stapling Instrument Having Separate Distinct Closing and Firing Systems", the disclosure of which is herein incorporated by reference in its entirety. Still other surgical stapling instruments may comprise, for example, open surgical staplers, linear surgical staplers, circular surgical staplers, etc.

Referring to FIGS. 1 and 2, an exemplary surgical stapling and severing instrument 10 incorporates an end effector 12 that has a second jaw 15 that is pivotally supported relative to a first jaw 13. For example, the first jaw 13 comprises an elongate channel 16 that operably supports a surgical staple cartridge 120 and the second jaw 15 comprises an anvil 100 that is movably attached to the elongate channel 16. The end effector 12 is coupled by a shaft 18 to a handle 20. An implement portion 22, formed by the end effector 12 and shaft 18, may be advantageously sized for insertion through a trocar or small laparoscopic opening to perform an endoscopic surgical procedure while being controlled by a surgeon grasping the handle 20. The handle 20 advantageously includes features that allow separate closure motion of the end effector 12 from firing, as well as enabling multiple firing strokes for applying a firing motion to effect firing (i.e., severing and stapling) of the end effector 12 while indicating the degree of firing to the surgeon.

To these ends, a closure tube 24 of the shaft 18 is coupled between a closure trigger 26 and the anvil 100 to cause closure of the end effector 12. Within the closure tube 24, a frame 28 is coupled between the elongate channel 16 and the handle 20 to longitudinally position and support the end effector 12. A rotation knob 30 is coupled with the frame 28, and both elements are rotatably coupled to the handle 20 with respect to a rotational movement about a longitudinal axis of the shaft 18. Such arrangement enables the surgeon to rotate the end effector 12 by turning the rotation knob 30. The closure tube 24 is also rotated by the rotation knob 30 but retains a degree of longitudinal movement relative thereto to cause the closure of the end effector 12. Within the frame 28, a firing rod 32 is positioned for longitudinal movement and coupled between the anvil 100 of the end effector 12 and a multiple-stroke firing trigger 34. The closure trigger 26 is distal to a pistol grip 36 of the handle 20 with the firing trigger 34 distal to both the pistol grip 36 and closure trigger 26.

In endoscopic operation, once the implement portion 22 is inserted into a patient to access a surgical site, a surgeon refers to an endoscopic or other diagnostic imaging device to position tissue between the anvil 100 and elongate channel 16. Grasping the closure trigger 26 and pistol grip 36, the surgeon may repeatedly grasp and position the tissue. Once satisfied as to the location of the tissue relative to the end effector 12 and the amount of tissue therein, the surgeon depresses the closure trigger 26 fully toward the pistol grip 36, clamping the tissue in the end effector 12 and locking the closure trigger 26 in this clamped (closed) position. If not satisfied with this position, the surgeon may release the closure trigger 26 by depressing a closure release button 38 and thereafter repeat the procedure to clamp tissue.

If clamping is correct, the surgeon may proceed with firing the surgical stapling and severing instrument 10. Specifically, the surgeon grasps the firing trigger 34 and pistol grip 36, depressing the firing trigger 34 a predetermined number of times. The number of firing strokes necessary is ergonomically determined based on a maximum hand size, maximum amount of force to be imparted to the instrument during each firing stroke, and the longitudinal distance and force needed to be transferred through the firing rod 32 to the end effector 12 during firing.

Other surgical stapling and severing instruments that may be employed include a motor-powered drive shaft arrangement for advancing and retracting a staple-driving sled and knife assembly. Examples of such instruments are disclosed in U.S. Patent No. 8,020,743, entitled "Powered Articulatable Surgical Cutting and Fastening Instrument With Flexible Drive Member", the entire disclosure of which is herein incorporated by reference. Robotically controlled surgical cutting and severing instruments may also be used, such as those disclosed in U.S. Patent Application entitled "Surgical Stapling Instruments With Rotatable Staple Deployment Arrangements", Serial No. 13/118,241, filed May 27, 2011, the entire disclosure of which is herein incorporated by reference.

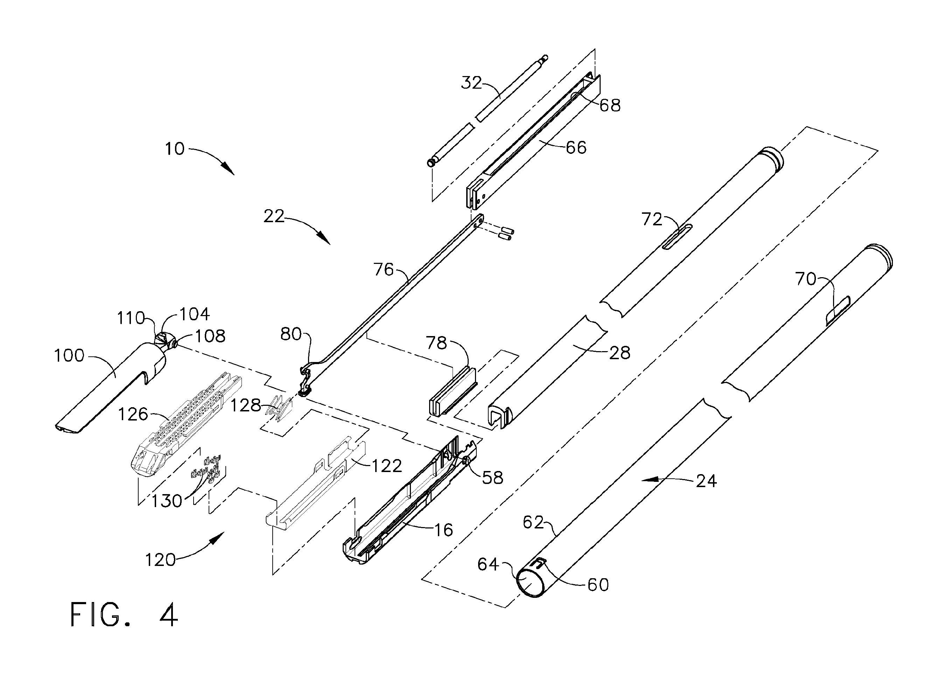

Referring now to FIG. 4, the implement portion 22 also includes components that respond to the firing motion of the firing rod 32. According to the invention, a distal end of the firing rod 32 rotatably engages a firing trough member 66 that has a longitudinal recess 68. Firing trough member 66 moves longitudinally within frame 28 in direct response to longitudinal motion of firing rod 32. A longitudinal slot 70 in the closure tube 24 operably couples with the rotation knob 30 and a short longitudinal slot 72 in the frame 28 radially aligned with the longitudinal slot 70 is engaged to the rotation knob 30. The length of the longitudinal slot 70 in the closure tube 24 is sufficiently long as to allow relative longitudinal motion with the rotation knob 30 to accomplish firing and closure motions respectively.

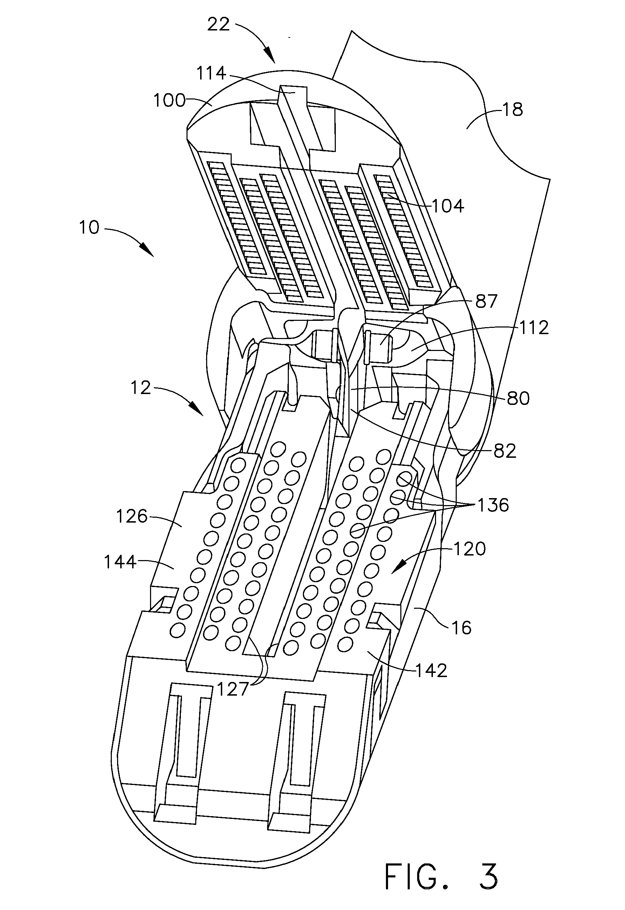

As illustrated, the distal end of the frame trough member 66 is attached to a proximal end of a firing bar 76 that moves with the frame 28, including a guide 78 therein, to distally project an E-beam 80 into the end effector 12. As indicated above, the end effector 12 includes a staple cartridge 120 that is actuated by the E-beam 80. The staple cartridge 120 has a tray 122 that holds a staple cartridge body 126, a wedge sled driver 128, staple drivers 130 and staples 132. It will be appreciated that the wedge sled driver 128 longitudinally moves within a recess 134 located between a cartridge tray 122 and the cartridge body 126. The wedge sled driver 128 presents camming surfaces that contact and lift the staple drivers 130 upward, driving the staples 132 up from staple apertures 136 into contact with staple forming pockets 104 in a staple forming surface 102 of the anvil 100, creating formed "B" shaped staples. With particular reference to FIG. 3, the staple cartridge body 126 further includes a proximally open, vertical deck slot 127 for passage of the E-beam 80. Cutting surface 82 is provided along a distal end of E-beam 80 to cut tissue after it is stapled.

In the depicted example, the anvil 100 responds to the closure motion from the handle 20 first by including an anvil mounting portion 105 that includes a pair of laterally projecting anvil trunnions 108 that are distal to a vertically projecting anvil feature 110 (FIG. 4). The anvil trunnions 108 translate within kidney shaped openings 58 in the elongate channel 16 to open and close anvil 100 relative to elongate channel 16. The anvil feature 56 engages a bent tab 59 (FIG. 2) extending inwardly in tab aperture 60 (FIG. 4) on a distal end 62 of the closure tube 24, the latter distally terminating in a distal edge 64 that pushes against the mounting portion 104. Thus, when the closure tube 24 moves proximally from its the open position, the bent tab 59 of the closure tube 24 draws the anvil feature 110 proximally, and the anvil pivot trunnions 108 follow the kidney shaped openings 58 of the channel 16 causing the anvil 100 to simultaneously translate proximally and rotate upward to the open position. When the closure tube 24 moves distally, the tab aperture 60 releases from the anvil feature 110 and the distal edge 64 pushes on the anvil mounting portion 104, closing the anvil 100.

Features of the E-beam 80 that facilitate firing of the end effector 12, in particular, are depicted. In FIG. 2, the wedge sled driver 128 is in its fully proximally position, indicating an unfired staple cartridge 120. A middle pin 83 is aligned to enter the firing recess 127 in the staple cartridge 120, for distally driving the wedge sled driver 128. A bottom pin or cap 85 of the E-beam 80 slides along a bottom surface of the elongate channel 16, thus the middle and bottom pins 83, 85 slidingly engage the elongate channel 16. In the open and unfired state of FIG. 2, top pins 87 of the E-beam 80 are residing within an anvil pocket 112 of the anvil 100, and thus does not impede repeated opening and closing of the anvil 100. When the end effector 12 is in the clamped and ready to fire state, the top pins 87 of the E-beam 80 are aligned with an anvil slot 114 in the anvil 100 distal to and communicating with the anvil pocket 112. When the end effector is fired, the E-beam 80 is advanced distally through the end effector cutting the tissue and firing the staples. As the E-beam 80 moves distally, the upper pins 87 translate down the anvil slot 114, affirmatively spacing the anvil 100 from the elongate channel 16 as the cutting surface 82 severs clamped tissue. Simultaneously, the middle pin 85 has actuated the staple cartridge 120. Thereafter, the E-beam 80 is retracted prior to opening the end effector 12 and replacing the staple cartridge 120 for an additional operation.

Optionally, as described above, a staple cartridge 120 can comprise a cartridge body 126 that has a plurality of staple cavities 140 therein. The cartridge body 126 can comprise a deck 142 that has a top deck surface 144 wherein each staple cavity 140 defines an opening in the deck surface 144. As also described above, a staple 132 is positioned within each staple cavity 140 such that the staples 132 are stored within the cartridge body 126 until they are ejected therefrom. Prior to being ejected from the cartridge body 126, the staples 132 can be contained with the cartridge body 126 such that the staples 132 do not protrude above the deck surface 144. As the staples 132 are positioned below the deck surface 144, In such cases, the possibility of the staples 132 becoming damaged and/or prematurely contacting the targeted tissue can be reduced. In various circumstances, the staples 132 can be moved between an unfired position in which they do not protrude from the cartridge body 126 and a fired position in which they have emerged from the cartridge body 126 and can contact an anvil 100 positioned opposite the staple cartridge 120. The anvil 100, and/or the forming pockets 104 defined within the anvil 100, can be positioned a predetermined distance above the deck surface 144 such that, as the staples 132 are being deployed from the cartridge body 126, the staples 132 are deformed to a predetermined formed height. In some circumstances, the thickness of the tissue captured between the anvil 100 and the staple cartridge 120 may vary and, as a result, thicker tissue may be captured within certain staples 132 while thinner tissue may be captured within certain other staples 132. In either event, the clamping pressure, or force, applied to the tissue by the staples 132 may vary from staple to staple or vary between a staple on one end of a staple row and a staple on the other end of the staple row, for example. In certain circumstances, the gap between the anvil 100 and the staple cartridge deck 142 can be controlled such that the staples 132 apply a certain minimum clamping pressure within each staple 132. In some such circumstances, however, significant variation of the clamping pressure within different staples may still exist.

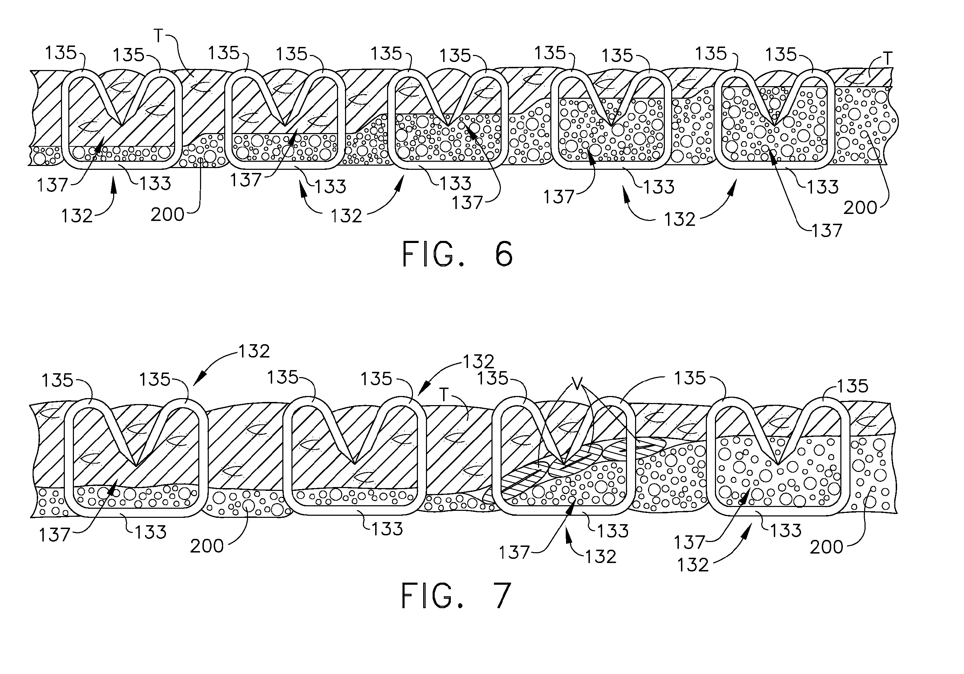

Referring primarily to FIG. 6 and as described in greater detail below, each staple 132 can comprise a base 133 and one or more legs 135 extending from the base 133. Prior to the staples 132 being deployed, the bases 133 of the staples 132 can be supported by staple drivers 130 positioned within the cartridge body 126 and, concurrently, the legs 135 of the staples 132 can be at least partially contained within the staple cavities 140.

Various means for compensating for the thickness of the tissue captured within the staples deployed from the staple cartridge are disclosed in U.S. Patent Application entitled "Tissue Thickness Compensator For a Surgical Stapler Comprising An Adjustable Anvil", Serial No. 13/097,891, filed April 29, 2011, the entire disclosure of which is herein incorporated by reference. A tissue thickness compensator 200 is employed. The staples 132 can be deployed between an unfired position and a fired position such that the legs 135 move through the tissue thickness compensator 200, penetrate through a top surface of the tissue thickness compensator 200, penetrate the tissue T, and contact the anvil 100 positioned opposite the staple cartridge 120. As the legs 135 are deformed against the anvil 100, the legs 135 of each staple 132 can capture a portion of the tissue thickness compensator 200 and a portion of the tissue T within each staple 132 and apply a compressive force to the tissue T. Further to the above, the legs 135 of each staple 132 can be deformed downwardly toward the base 133 of the staple 132 to form a staple entrapment area 137 in which the tissue T and the tissue thickness compensator 200 can be captured. In various circumstances, the staple entrapment area 137 can be defined between the inner surfaces of the deformed legs 135 and the inner surface of the base 133. The size of the entrapment area for a staple can depend on several factors such as the length of the legs, the diameter of the legs, the width of the base, and/or the extent in which the legs are deformed, for example.

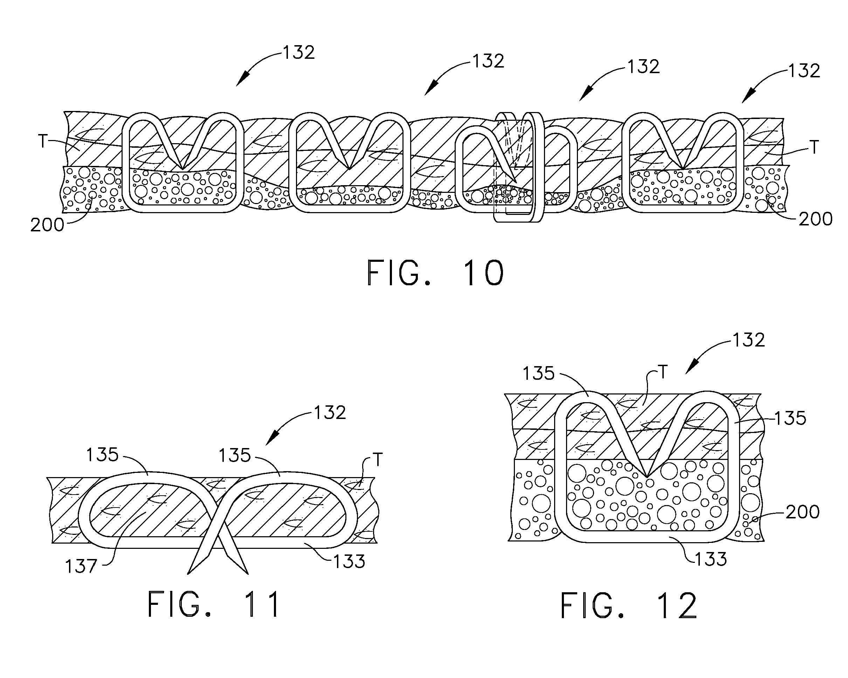

Previously, a surgeon was often required to select the appropriate staples having the appropriate staple height for the tissue being stapled. For example, a surgeon could select tall staples for use with thick tissue and short staples for use with thin tissue. In some circumstances, however, the tissue being stapled did not have a consistent thickness and, thus, some staples were unable to achieve the desired fired configuration. FIG. 11 illustrates a tall staple used in thin tissue. Referring now to FIG. 12, when a tissue thickness compensator such as a tissue thickness compensator 200, for example, is used within thin tissue, the staple may be formed to a desired fired configuration.

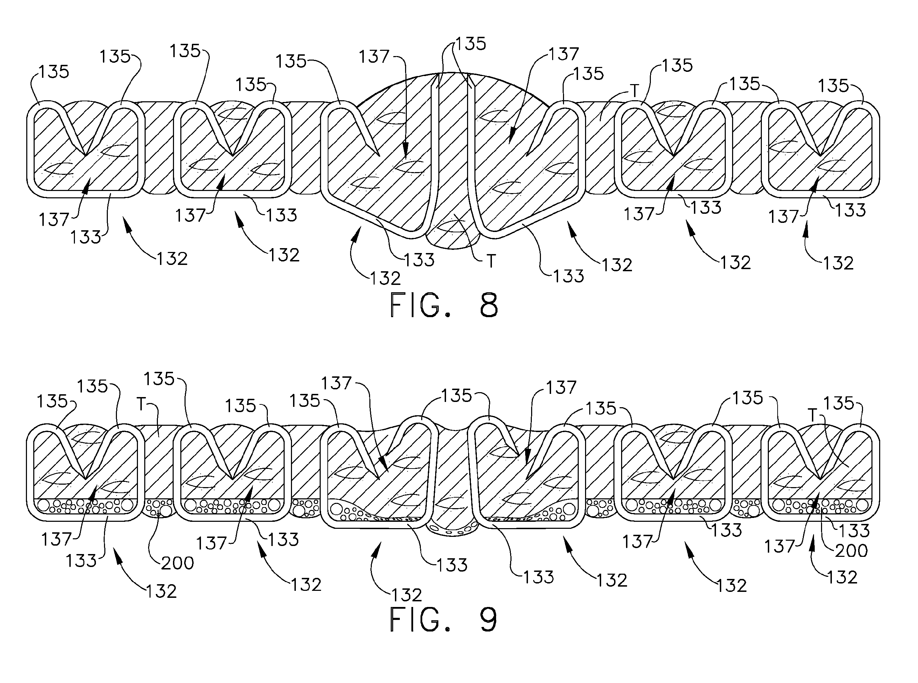

Owing to the compressibility of the tissue thickness compensator, the tissue thickness compensator can compensate for the thickness of the tissue captured within each staple. More particularly, referring now to FIGS. 6 and 7, a tissue thickness compensator, such as tissue thickness compensator 200, for example, can consume larger and/or smaller portions of the staple entrapment area 137 of each staple 132 depending on the thickness and/or type of tissue contained within the staple entrapment area 137. For example, if thinner tissue T is captured within a staple 132, the tissue thickness compensator 200 can consume a larger portion of the staple entrapment area 137 as compared to circumstances where thicker tissue T is captured within the staple 132. Correspondingly, if thicker tissue T is captured within a staple 132, the tissue thickness compensator 200 can consume a smaller portion of the staple entrapment area 137 as compared to the circumstances where thinner tissue T is captured within the staple 132. In this way, the tissue thickness compensator 200 can compensate for thinner tissue and/or thicker tissue and assure that a compressive pressure is applied to the tissue irrespective, or at least substantially irrespective, of the tissue thickness captured within the staples. In addition to the above, the tissue thickness compensator 200 can compensate for different types, or compressibilities, of tissues captured within different staples 132. Referring now to FIG. 7, the tissue thickness compensator 200 can apply a compressive force to vascular tissue T which can include vessels V and, as a result, restrict the flow of blood through the less compressible vessels V while still applying a desired compressive pressure to the surrounding tissue T. In various circumstances, further to the above, the tissue thickness compensator 200 can also compensate for malformed staples. Referring to FIG. 8, the malformation of various staples 132 can result in larger staple entrapment areas 137 being defined within such staples. Owing to the resiliency of the tissue thickness compensator 200, referring now to FIG. 9, the tissue thickness compensator 200 positioned within malformed staples 132 may still apply a sufficient compressive pressure to the tissue T even though the staple entrapment areas 137 defined within such malformed staples 132 may be enlarged. In various circumstances, the tissue thickness compensator 200 located intermediate adjacent staples 132 can be biased against the tissue T by properly-formed staples 132 surrounding a malformed staple 132 and, as a result, apply a compressive pressure to the tissue surrounding and/or captured within the malformed staple 132, for example. In various circumstances, a tissue thickness compensator can compensate for different tissue densities which can arise due to calcifications, fibrous areas, and/or tissue that has been previously stapled or treated, for example.

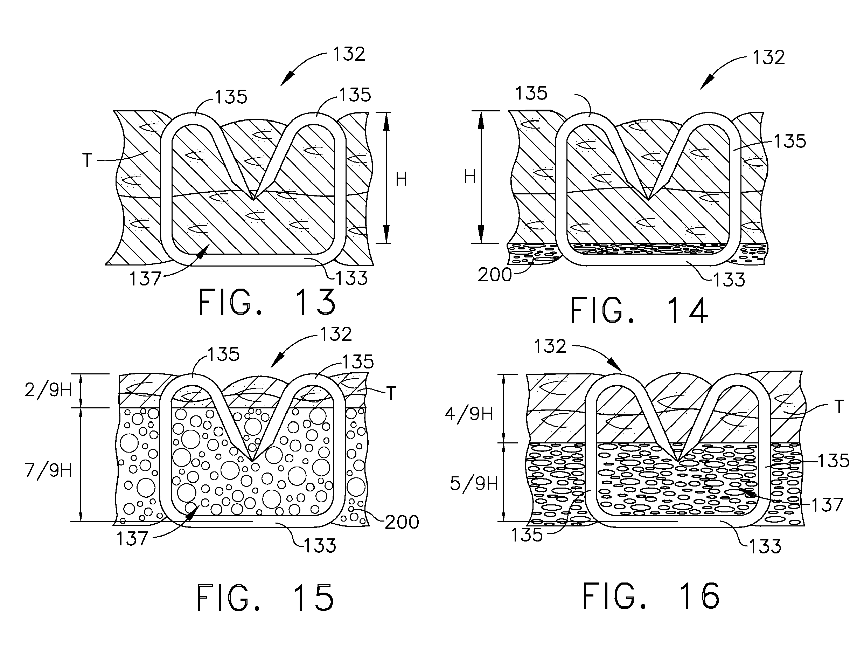

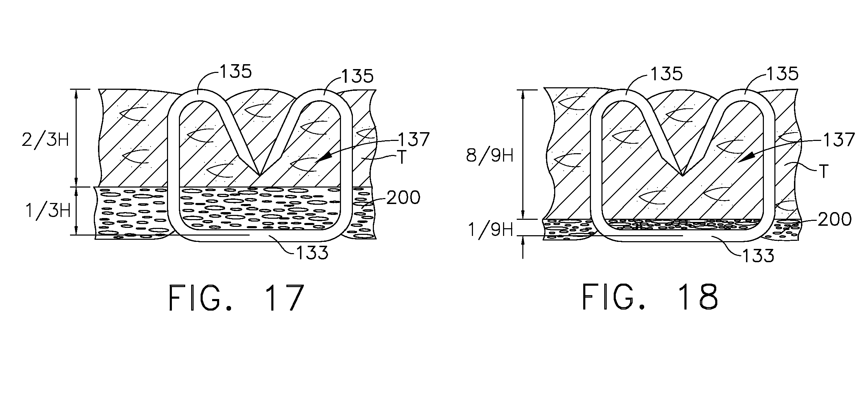

According to the invention, a fixed, or unchangeable, tissue gap can be defined between the support portion and the anvil and, as a result, the staples may be deformed to a predetermined height regardless of the thickness of the tissue captured within the staples. When a tissue thickness compensator is used in such cases, the tissue thickness compensator can adapt to the tissue captured between the anvil and the support portion staple cartridge and, owing to the resiliency of the tissue thickness compensator, the tissue thickness compensator can apply an additional compressive pressure to the tissue. Referring now to FIGS. 13-18, a staple 132 has been formed to a predefined height H. With regard to FIG. 13, a tissue thickness compensator has not been utilized and the tissue T consumes the entirety of the staple entrapment area 137. With regard to FIG. 14, a portion of a tissue thickness compensator 200 has been captured within the staple 132, compressed the tissue T, and consumed at least a portion of the staple entrapment area 137. Referring now to FIG. 15, thin tissue T has been captured within the staple 132. In this embodiment, the compressed tissue T has a height of approximately 2/9H and the compressed tissue thickness compensator 200 has a height of approximately 7/9H, for example. Referring now to FIG. 16, tissue T having an intermediate thickness has been captured within the staple 132. In this embodiment, the compressed tissue T has a height of approximately 4/9H and the compressed tissue thickness compensator 200 has a height of approximately 5/9H, for example. Referring now to FIG. 17, tissue T having an intermediate thickness has been captured within the staple 132. In this embodiment, the compressed tissue T has a height of approximately 2/3H and the compressed tissue thickness compensator 200 has a height of approximately 1/3H, for example. Referring now to FIG. 18, thick tissue T has been captured within the staple 132. In this embodiment, the compressed tissue T has a height of approximately 8/9H and the compressed tissue thickness compensator 200 has a height of approximately 1/9H, for example. In various circumstances, the tissue thickness compensator can comprise a compressed height which comprises approximately 10% of the staple entrapment height, approximately 20% of the staple entrapment height, approximately 30% of the staple entrapment height, approximately 40% of the staple entrapment height, approximately 50% of the staple entrapment height, approximately 60% of the staple entrapment height, approximately 70% of the staple entrapment height, approximately 80% of the staple entrapment height, and/or approximately 90% of the staple entrapment height, for example.

The staples 132 can comprise any suitable unformed height. The staples 132 can comprise an unformed height between approximately 2 mm and approximately 4.8 mm, for example. The staples 132 can comprise an unformed height of approximately 2.0 mm, approximately 2.5 mm, approximately 3.0 mm, approximately 3.4 mm, approximately 3.5 mm, approximately 3.8 mm, approximately 4.0 mm, approximately 4.1 mm, and/or approximately 4.8 mm, for example. The height H to which the staples can be deformed can be dictated by the distance between the deck surface 144 of the staple cartridge 126 and the opposing anvil 100. The distance between the deck surface 144 and the staple forming surface 102 of the anvil 100 can be approximately 0.097", for example. The height H can also be dictated by the depth of the forming pockets defined within the anvil. The forming pockets can have a depth measured from the tissue-contacting surface, for example.

As described above, the staple cartridge 120 includes staple drivers 130 which can lift the staples 132 toward the anvil 100 and, lift, or "overdrive", the staples above the deck surface 144. In such cases, the height H to which the staples 132 are formed can also be dictated by the distance in which the staples 132 are overdriven. For example, the staples 132 can be overdriven by approximately .028", for example, and can result in the staples 132 being formed to a height of approximately 0.189", for example. The staples 132 can be formed to a height of approximately 0.8 mm, approximately 1.0 mm, approximately 1.5 mm, approximately 1.8 mm, approximately 2.0 mm, and/or approximately 2.25 mm, for example. The staples 132 can be formed to a height between approximately 2.25 mm and approximately 3.0 mm, for example. Further to the above, the height of the staple entrapment area of a staple can be determined by the formed height of the staple and the width, or diameter, of the wire comprising the staple. The height of the staple entrapment area 137 of a staple 132 can comprise the formed height H of the staple less two diameter widths of the wire. The staple wire can comprise a diameter of approximately 0.0089", for example. The staple wire can comprise a diameter between approximately 0.0069" and approximately 0.0119", for example. For example, the formed height H of a staple 10030 can be approximately 0.189" and the staple wire diameter can be approximately 0.0089" resulting in a staple entrapment height of approximately 0.171 ", for example.

Further to the above, the tissue thickness compensator can comprise an uncompressed, or pre-deployed, height and can be configured to deform to one of a plurality of compressed heights. The tissue thickness compensator can comprise an uncompressed height of approximately 0.125", for example. The tissue thickness compensator can comprise an uncompressed height of greater than or equal to approximately 0.080", for example. The tissue thickness compensator can comprise an uncompressed, or pre-deployed, height which is greater than the unfired height of the staples. The uncompressed, or pre-deployed, height of the tissue thickness compensator can be approximately 10% taller, approximately 20% taller, approximately 30% taller, approximately 40% taller, approximately 50% taller, approximately 60% taller, approximately 70% taller, approximately 80% taller, approximately 90% taller, and/or approximately 100% taller than the unfired height of the staples, for example. The uncompressed, or pre-deployed, height of the tissue thickness compensator can be up to approximately 100% taller than the unfired height of the staples, for example. The uncompressed, or pre-deployed, height of the tissue thickness compensator can be over 100% taller than the unfired height of the staples, for example. The tissue thickness compensator can comprise an uncompressed height which is equal to the unfired height of the staples. The tissue thickness compensator can comprise an uncompressed height which is less than the unfired height of the staples. The uncompressed, or pre-deployed, height of the thickness compensator can be approximately 10% shorter, approximately 20% shorter, approximately 30% shorter, approximately 40% shorter, approximately 50% shorter, approximately 60% shorter, approximately 70% shorter, approximately 80% shorter, and/or approximately 90% shorter than the unfired height of the staples, for example. The compressible second portion can comprise an uncompressed height which is taller than an uncompressed height of the tissue T being stapled. The tissue thickness compensator can comprise an uncompressed height which is equal to an uncompressed height of the tissue T being stapled. The tissue thickness compensator can comprise an uncompressed height which is shorter than an uncompressed height of the tissue T being stapled.

As described above, a tissue thickness compensator can be compressed within a plurality of formed staples regardless of whether thick tissue or thin tissue is captured within the staples. For example, the staples within a staple line, or row, can be deformed such that the staple entrapment area of each staple comprises a height of approximately 2.0 mm, for example, wherein the tissue T and the tissue thickness compensator can be compressed within this height. In certain circumstances, the tissue T can comprise a compressed height of approximately 1.75 mm within the staple entrapment area while the tissue thickness compensator can comprise a compressed height of approximately 0.25 mm within the staple entrapment area, thereby totaling the approximately 2.0 mm staple entrapment area height, for example. In certain circumstances, the tissue T can comprise a compressed height of approximately 1.50 mm within the staple entrapment area while the tissue thickness compensator can comprise a compressed height of approximately 0.50 mm within the staple entrapment area, thereby totaling the approximately 2.0 mm staple entrapment area height, for example. In certain circumstances, the tissue T can comprise a compressed height of approximately 1.25 mm within the staple entrapment area while the tissue thickness compensator can comprise a compressed height of approximately 0.75 mm within the staple entrapment area, thereby totaling the approximately 2.0 mm staple entrapment area height, for example. In certain circumstances, the tissue T can comprise a compressed height of approximately 1.0 mm within the staple entrapment area while the tissue thickness compensator can comprise a compressed height of approximately 1.0 mm within the staple entrapment area, thereby totaling the approximately 2.0 mm staple entrapment area height, for example. In certain circumstances, the tissue T can comprise a compressed height of approximately 0.75 mm within the staple entrapment area while the tissue thickness compensator can comprise a compressed height of approximately 1.25 mm within the staple entrapment area, thereby totaling the approximately 2.0 mm staple entrapment area height, for example. In certain circumstances, the tissue T can comprise a compressed height of approximately 1.50 mm within the staple entrapment area while the tissue thickness compensator can comprise a compressed height of approximately 0.50 mm within the staple entrapment area, thereby totaling the approximately 2.0 mm staple entrapment area height, for example. In certain circumstances, the tissue T can comprise a compressed height of approximately 0.25 mm within the staple entrapment area while the tissue thickness compensator can comprise a compressed height of approximately 1.75 mm within the staple entrapment area, thereby totaling the approximately 2.0 mm staple entrapment area height, for example.

Further to the above, the tissue thickness compensator can comprise an uncompressed height which is less than the fired height of the staples. The tissue thickness compensator can comprise an uncompressed height which is equal to the fired height of the staples. The tissue thickness compensator can comprise an uncompressed height which is taller than the fired height of the staples. For example, the uncompressed height of a tissue thickness compensator can comprise a thickness which is approximately 110% of the formed staple height, approximately 120% of the formed staple height, approximately 130% of the formed staple height, approximately 140% of the formed staple height, approximately 150% of the formed staple height, approximately 160% of the formed staple height, approximately 170% of the formed staple height, approximately 180% of the formed staple height, approximately 190% of the formed staple height, and/or approximately 200% of the formed staple height, for example. The tissue thickness compensator can comprise an uncompressed height which is more than twice the fired height of the staples. The tissue thickness compensator can comprise a compressed height which is from approximately 85% to approximately 150% of the formed staple height, for example. Optionally, as described above, the tissue thickness compensator can be compressed between an uncompressed thickness and a compressed thickness. The compressed thickness of a tissue thickness compensator can be approximately 10% of its uncompressed thickness, approximately 20% of its uncompressed thickness, approximately 30% of its uncompressed thickness, approximately 40% of its uncompressed thickness, approximately 50% of its uncompressed thickness, approximately 60% of its uncompressed thickness, approximately 70% of its uncompressed thickness, approximately 80% of its uncompressed thickness, and/ or approximately 90% of its uncompressed thickness, for example. The uncompressed thickness of the tissue thickness compensator can be approximately two times, approximately ten times, approximately fifty times, and/or approximately one hundred times thicker than its compressed thickness, for example. The compressed thickness of the tissue thickness compensator can be between approximately 60% and approximately 99% of its uncompressed thickness. The uncompressed thickness of the tissue thickness compensator can be at least 50% thicker than its compressed thickness. The uncompressed thickness of the tissue thickness compensator can be up to one hundred times thicker than its compressed thickness. The compressible second portion can be elastic, or at least partially elastic, and can bias the tissue T against the deformed legs of the staples. For example, the compressible second portion can resiliently expand between the tissue T and the base of the staple in order to push the tissue T against the legs of the staple. As discussed in further detail below, the tissue thickness compensator can be positioned intermediate the tissue T and the deformed staple legs. In various circumstances, as a result of the above, the tissue thickness compensator can be configured to consume any gaps within the staple entrapment area.

The tissue thickness compensator may comprise a polymeric composition. The polymeric composition may comprise one or more synthetic polymer and/or one or more non-synthetic polymer. The synthetic polymer may comprise a synthetic absorbable polymer and/or a synthetic non-absorbable polymer. The polymeric composition may comprise a biocompatible foam, for example. The biocompatible foam may comprise a porous, open cell foam and/or a porous, closed cell foam, for example. The biocompatible foam can have a uniform pore morphology or may have a gradient pore morphology (i.e. small pores gradually increasing in size to large pores across the thickness of the foam in one direction). The polymeric composition may comprise one or more of a porous scaffold, a porous matrix, a gel matrix, a hydrogel matrix, a solution matrix, a filamentous matrix, a tubular matrix, a composite matrix, a membranous matrix, a biostable polymer, and a biodegradable polymer, and combinations thereof For example, the tissue thickness compensator may comprise a foam reinforced by a filamentous matrix or may comprise a foam having an additional hydrogel layer that expands in the presence of bodily fluids to further provide the compression on the tissue. According to the invention, a tissue thickness compensator could also be comprised of a coating on a material and/or a second or third layer that expands in the presence of bodily fluids to further provide the compression on the tissue. Such a layer could be a hydrogel that could be a synthetic and/or naturally derived material and could be either biodurable and/or biodegradable, for example. A tissue thickness compensator could be reinforced with fibrous non-woven materials or fibrous mesh type elements, for example, that can provide additional flexibility, stiffness, and/or strength. According to the invention, a tissue thickness compensator that has a porous morphology which exhibits a gradient structure such as, for example, small pores on one surface and larger pores on the other surface. Such morphology could be more optimal for tissue in-growth or hemostatic behavior. Further, the gradient could be also compositional with a varying bio-absorption profile. A short term absorption profile may be preferred to address hemostasis while a long term absorption profile may address better tissue healing without leakages.

Examples of non-synthetic polymers include, but are not limited to, lypholized polysaccharide, glycoprotein, elastin, proteoglycan, gelatin, collagen, and oxidized regenerated cellulose (ORC). Examples of synthetic absorbable polymers include, but are not limited to, poly(lactic acid) (PLA), poly(L-lactic acid) (PLLA), polycaprolactone (PCL), polyglycolic acid (PGA), poly(trimethylene carbonate) (TMC), polyethylene terephthalate (PET), polyhydroxyalkanoate (PHA), a copolymer of glycolide and ε-caprolactone (PGCL), a copolymer of glycolide and-trimethylene carbonate, poly(glycerol sebacate) (PGS), polydioxanone, poly(orthoesters), polyanhydrides, polysaccharides, poly(ester-amides), tyrosine-based polyarylates, tyrosine-based polyiminocarbonates, tyrosine-based polycarbonates, poly(D,L-lactide-urethane), poly(B-hydroxybutyrate), poly(E-caprolactone), polyethyleneglycol (PEG), poly[bis(carboxylatophenoxy) phosphazene], poly(amino acids), pseudo-poly(amino acids), absorbable polyurethanes, and combinations thereof. The polymeric composition may comprise from approximately 50% to approximately 90% by weight of the polymeric composition of PLLA and approximately 50% to approximately 10% by weight of the polymeric composition of PCL, for example. The polymeric composition may comprise approximately 70% by weight of PLLA and approximately 30% by weight of PCL, for example. The polymeric composition may comprise from approximately 55% to approximately 85% by weight of the polymeric composition of PGA and 15% to 45% by weight of the polymeric composition of PCL, for example. The polymeric composition may comprise approximately 65% by weight of PGA and approximately 35% by weight of PCL, for example. The polymeric composition may comprise from approximately 90% to approximately 95% by weight of the polymeric composition of PGA and approximately 5% to approximately 10% by weight of the polymeric composition of PLA, for example.

The synthetic absorbable polymer may comprise a bioabsorbable, biocompatible elastomeric copolymer. Suitable bioabsorbable, biocompatible elastomeric copolymers include but are not limited to copolymers of epsilon-caprolactone and glycolide (preferably having a mole ratio of epsilon-caprolactone to glycolide of from about 30:70 to about 70:30, preferably 35:65 to about 65:35, and more preferably 45:55 to 35:65); elastomeric copolymers of epsilon-caprolactone and lactide, including L-lactide, D-lactide blends thereof or lactic acid copolymers (preferably having a mole ratio of epsilon-caprolactone to lactide of from about 35:65 to about 65:35 and more preferably 45:55 to 30:70) elastomeric copolymers of p-dioxanone (1,4-dioxan-2-one) and lactide including L-lactide, D-lactide and lactic acid (preferably having a mole ratio of p-dioxanone to lactide of from about 40:60 to about 60:40); elastomeric copolymers of epsilon-caprolactone and p-dioxanone (preferably having a mole ratio of epsilon-caprolactone to p-dioxanone of from about 30:70 to about 70:30); elastomeric copolymers of p-dioxanone and trimethylene carbonate (preferably having a mole ratio of p-dioxanone to trimethylene carbonate of from about 30:70 to about 70:30); elastomeric copolymers of trimethylene carbonate and glycolide (preferably having a mole ratio of trimethylene carbonate to glycolide of from about 30:70 to about 70:30); elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof. The elastomeric copolymer may be a copolymer of glycolide and epsilon-caprolactone. Alternatively, the elastomeric copolymer is a copolymer of lactide and epsilon-caprolactone.

The disclosures of U.S. Patent No. 5,468,253, entitled ELASTOMERIC MEDICAL DEVICE, which issued on November 21, 1995, and U.S. Patent No. 6,325,810, entitled FOAM BUTTRESS FOR STAPLING APPARATUS, which issued on December 4, 2001, are hereby incorporated by reference in their respective entireties.

The synthetic absorbable polymer may comprise one or more of 90/10 poly(glycolide-L-lactide) copolymer, commercially available from Ethicon, Inc. under the trade designation VICRYL (polyglactic 910), polyglycolide, commercially available from American Cyanamid Co. under the trade designation DEXON, polydioxanone, commercially available from Ethicon, Inc. under the trade designation PDS, poly(glycolide-trimethylene carbonate) random block copolymer, commercially available from American Cyanamid Co. under the trade designation MAXON, 75/25 poly(glycolide-E-caprolactone-poliglecaprolactone 25) copolymer, commercially available from Ethicon under the trade designation MONOCRYL, for example.

Examples of synthetic non-absorbable polymers include, but are not limited to, foamed polyurethane, polypropylene (PP), polyethylene (PE), polycarbonate, polyamides, such as nylon, polyvinylchloride (PVC), polymethylmetacrylate (PMMA), polystyrene (PS), polyester, polyetheretherketone (PEEK), polytetrafluoroethylene (PTFE), polytrifluorochloroethylene (PTFCE), polyvinylfluoride (PVF), fluorinated ethylene propylene (FEP), polyacetal, polysulfone, and combinations thereof. The synthetic non-absorbable polymers may include, but are not limited to, foamed elastomers and porous elastomers, such as, for example, silicone, polyisoprene, and rubber. The synthetic polymers may comprise expanded polytetrafluoroethylene (ePTFE), commercially available from W. L. Gore & Associates, Inc. under the trade designation GORE-TEX Soft Tissue Patch and co-polyetherester urethane foam commercially available from Polyganics under the trade designation NASOPORE.

The polymeric composition of a tissue thickness compensator may be characterized by percent porosity, pore size, and/or hardness, for example. The polymeric composition may have a percent porosity from approximately 30% by volume to approximately 99% by volume, for example. The polymeric composition may have a percent porosity from approximately 60% by volume to approximately 98% by volume, for example. The polymeric composition may have a percent porosity from approximately 85% by volume to approximately 97% by volume, for example. The polymeric composition may comprise approximately 70% by weight of PLLA and approximately 30% by weight of PCL, for example, and can comprise approximately 90% porosity by volume, for example. For example, as a result, the polymeric composition would comprise approximately 10% copolymer by volume. The polymeric composition may comprise approximately 65% by weight of PGA and approximately 35% by weight of PCL, for example, and can have a percent porosity from approximately 93% by volume to approximately 95% by volume, for example. The polymeric composition may comprise a greater than 85% porosity by volume. The polymeric composition may have a pore size from approximately 5 micrometers to approximately 2000 micrometers, for example. The polymeric composition may have a pore size between approximately 10 micrometers to approximately 100 micrometers, for example. For example, the polymeric composition can comprise a copolymer of PGA and PCL, for example. The polymeric composition may have a pore size between approximately 100 micrometers to approximately 1000 micrometers, for example. For example, the polymeric composition can comprise a copolymer of PLLA and PCL, for example. According to certain aspects, the hardness of a polymeric composition may be expressed in terms of the Shore Hardness, which can defined as the resistance to permanent indentation of a material as determined with a durometer, such as a Shore Durometer. In order to assess the durometer value for a given material, a pressure is applied to the material with a durometer indenter foot in accordance with ASTM procedure D2240-00, entitled, "Standard Test Method for Rubber Property-Durometer Hardness", the entirety of which is incorporated herein by reference. The durometer indenter foot may be applied to the material for a sufficient period of time, such as 15 seconds, for example, wherein a reading is then taken from the appropriate scale. Depending on the type of scale being used, a reading of 0 can be obtained when the indenter foot completely penetrates the material, and a reading of 100 can be obtained when no penetration into the material occurs. This reading is dimensionless. The durometer may be determined in accordance with any suitable scale, such as Type A and/or Type OO scales, for example, in accordance with ASTM D2240-00. The polymeric composition of a tissue thickness compensator may have a Shore A hardness value from approximately 4 A to approximately 16 A, for example, which is approximately 45 OO to approximately 65 OO on the Shore OO range. For example, the polymeric composition can comprise a PLLA/PCL copolymer or a PGA/PCL copolymer, for example. The polymeric composition of a tissue thickness compensator may have a Shore A Hardness value of less than 15 A. The polymeric composition of a tissue thickness compensator may have a Shore A Hardness value of less than 10 A. The polymeric composition of a tissue thickness compensator may have a Shore A Hardness value of less than 5 A. The polymeric material may have a Shore OO composition value from approximately 35 OO to approximately 75 OO, for example.

The polymeric composition may have at least two of the above-identified properties. The polymeric composition may have at least three of the above-identified properties. The polymeric composition may have a porosity from 85% to 97% by volume, a pore size from 5 micrometers to 2000 micrometers, and a Shore A hardness value from 4 A to 16 A and Shore OO hardness value from 45 OO to 65 OO, for example. The polymeric composition may comprise 70% by weight of the polymeric composition of PLLA and 30% by weight of the polymeric composition of PCL having a porosity of 90% by volume, a pore size from 100 micrometers to 1000 micrometers, and a Shore A hardness value from 4 A to 16 A and Shore OO hardness value from 45 OO to 65 OO , for example. The polymeric composition may comprise 65% by weight of the polymeric composition of PGA and 35% by weight of the polymeric composition of PCL having a porosity from 93% to 95% by volume, a pore size from 10 micrometers to 100 micrometers, and a Shore A hardness value from 4 A to 16 A and Shore OO hardness value from 45 OO to 65 OO, for example.

The polymeric composition may comprise a pharmaceutically active agent. The polymeric composition may release a therapeutically effective amount of the pharmaceutically active agent. The pharmaceutically active agent may be released as the polymeric composition is desorbed/absorbed. The pharmaceutically active agent may be released into fluid, such as, for example, blood, passing over or through the polymeric composition. Examples of pharmaceutically active agents may include, but are not limited to, hemostatic agents and drugs, such as, for example, fibrin, thrombin, and oxidized regenerated cellulose (ORC); antiinflammatory drugs, such as, for example, diclofenac, aspirin, naproxen, sulindac, and hydrocortisone; antibiotic and antimicrobial drug or agents, such as, for example, triclosan, ionic silver, ampicillin, gentamicin, polymyxin B, chloramphenicol; and anticancer agents, such as, for example, cisplatin, mitomycin, adriamycin. As used herein, the term "tissue thickness compensator" may comprise any of the compensator compositions described above.

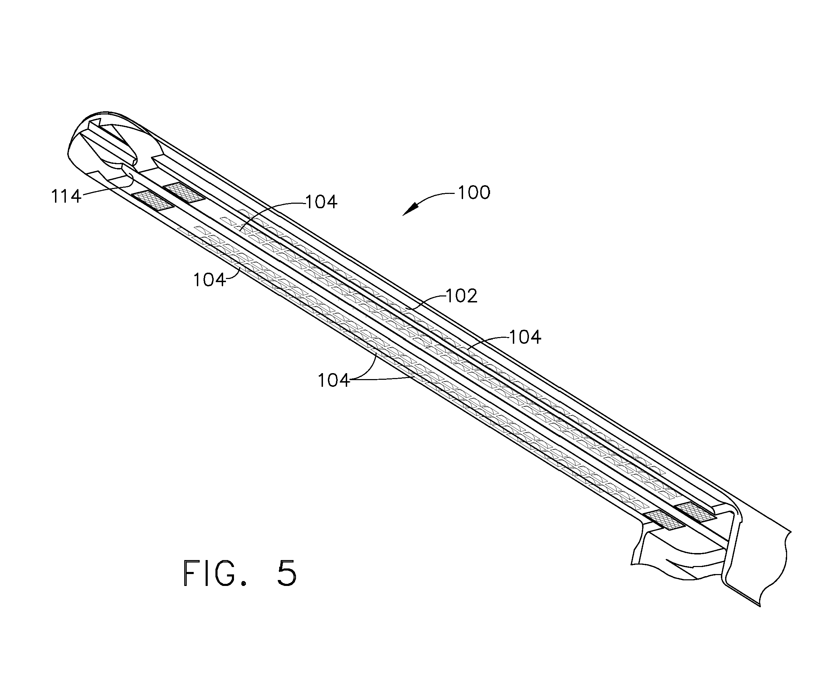

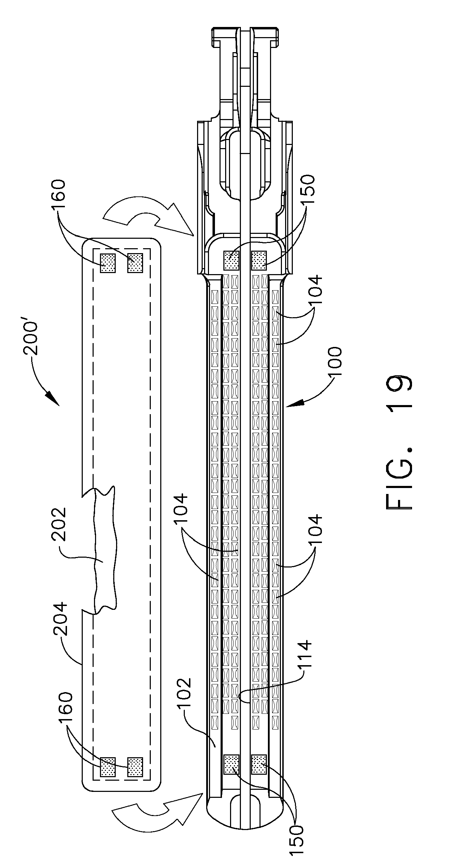

Arrangements for removably attaching various tissue thickness compensators to one of the first and secondjaws 13, 15 of a surgical stapling instrument are disclosed. For example, FIGS. 5 and 19 illustrate a staple forming under surface 102 of an anvil 100 that incorporates at least one area 150 of attachment protrusions for removably attaching a thickness compensator to the staple forming surface 102 of the anvil 100. For example, the area 150 of attachment protrusions may comprise the entire staple forming surface 102 not occupied by the staple forming pockets 104 formed therein or some lesser percentage of such surface 102. In FIGS. 5 and 19, for example, four discrete areas 150 of attachment protrusions are provided on the staple forming under surface 102 of the anvil 100 which are designed to removably mate with corresponding four areas 160 of attachment protrusions provided on a thickness compensator 200'.

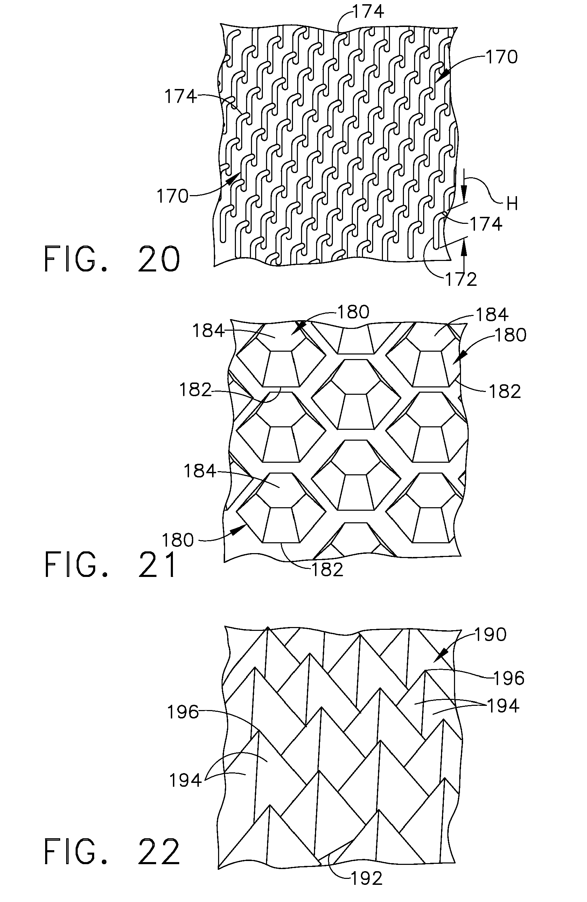

The thickness compensator 200' comprises a compressible body or foam member 202 that may comprise any of the various foam compositions/configurations described above. The compressible body 202 may carry a biological material such as, for example, oxidized regenerated cellulose "ORC", Fibrin, Thrombin, Non-woven absorbable polymer strands, platelet-rich plasma, calcium & albumin, collagen, hyaluronic acid, etc. The foam member 202 is encased or sealed or protected from fluid by a film 204. The film 204 may comprise a film fabricated from 65/35PCL/PGA. However, the film may be fabricated from any of the absorbable polymers or their copolymers described above. As can be seen in FIG. 19 corresponding areas 160 of attachment protrusions are attached to the film 204 and are configured to removably mate with the areas 150 of attachment protrusions on the anvil 100. The areas 150, 160 may comprise areas of corresponding hook and loop members commercially sold under the trademark VELCRO®. Alternatively, the areas 150, 160 may comprise protrusions 170 that have a protruding body portion 172 that has a distal end 174 that is not substantially coaxially aligned with the body portion 172. For example, the protrusions 170 may be somewhat hook-shaped. See FIG. 20. The height "H" of each protrusion 170 may be substantially greater than its cross-sectional area. The protrusions 170 may be fabricated from, for example, absorbable polymer material of the types described above such as, for example, PGA, PCL, PLA, PEO (Polyethyne oxide), TMC, DMTMC. However, the protrusions may also be fabricated from, for example, regular Nylon, polycarbonate, Ultem or polyethylene if they stay with the device and are not implanted. Such protrusions 170 may, for example, be provided in a density of approximately, 130 to 1700 protrusions per square inch of area such that when the areas 150 and 160 are brought into mating engagement, the thickness compensator 200' is retainingly affixed to the staple forming surface 102 of the anvil 100. However, the protrusions 170 may be fabricated from other suitable materials and provided in other suitable densities that serve to removably affix the tissue thickness compensator 200' to the anvil 100. Thus, the term "areas of attachment protrusion" is intended to encompass at least one attachment protrusion configured to releasably engage a tissue thickness compensator as well as plural attachment protrusions arrangements of various densities.

Alternatively, the areas 150, 160 may comprise protrusions 180 that have a substantially hexagonal shape. As can be seen in FIG. 21, for example, each protrusion 180 tapers from its base 182 to a hexagonally-shaped distal end 184. The protrusions 180 may be fabricated from, for example, absorbable polymer material of the types described above such as, for example, PGA, PCL, PLA, PEO (Polyethyne oxide), TMC, DMTMC. However, the protrusions may also be fabricated from, for example, regular Nylon, polycarbonate, Ultem or polyethylene if they stay with the device and are not implanted. Such protrusions 180 may, for example, be provided in a density of approximately, 130-1700 protrusions per square inch of area such that when the areas 150 and 160 are brought into mating engagement, the thickness compensator 200' is retainingly affixed to the staple forming surface 102 of the anvil 100. However, the protrusions 180 may be fabricated from other suitable materials and provided in other suitable densities that serve to removably affix the tissue thickness compensator 200 to the anvil 100.

Alternatively, the areas 150, 160 may comprise protrusions 190 that are substantially pyramidal in shape. As can be seen in FIG. 22, for example, each protrusion 190 has four substantially triangular-shaped sides 194 that taper from a base 192 to a pointed distal end 196. The protrusions 180 may be fabricated from, for example, absorbable polymer material of the types described above such as, for example, PGA, PCL, PLA, PEO (Polyethyne oxide), TMC, DMTMC. However, the protrusions may also be fabricated from, for example, regular Nylon, polycarbonate, Ultem or polyethylene if they stay with the device and are not implanted. Such protrusions 180 may, for example, be provided in a density of approximately, 130-1700 protrusions per square inch of area such that when the areas 150 and 160 are brought into mating engagement, the thickness compensator 200' is retainingly affixed to the staple forming surface 102 of the anvil 100. However, the protrusions 180 may be fabricated from other suitable materials and provided in other suitable densities that serve to removably affix the tissue thickness compensator 200' to the anvil 100.

The various embodiments of attachment protrusions employed serve to removably attach the tissue compensator 200' to the anvil 100 without employing adhesives as the sole medium of attachment which are generally ill-suited to facilitate removable attachment of the tissue compensator 200' to the anvil. As an additional alternative, adhesion between the areas 150 and 160 may be improved by adding polystyrene particles into the protrusions. They also serve to avoid obstructing the slot 114 in the anvil 100 with means for attaching the tissue thickness compensator 200 to the anvil 100. In any event, once the end effector 12 has been fired and the tissue thickness compensator has been cut and stapled to the target tissue, the areas of attachment protrusions 150, 160 facilitate detachment of the end effector 12 from the tissue thickness compensator 200.

Alternatively, the area(s) 150 of attachment protrusions may be integrally formed in the anvil 100. As an additional alternative, the area(s) 150 of attachment protrusions are provided on the deck surface 144 of the staple cartridge 120 and are configured to retainingly mate with the corresponding area(s) 160 of protrusions on the tissue thickness compensator 200'. Alternatively, the area(s) 150 of protrusions may be integrally formed in the deck 142. The area(s) 150 may be located so as to avoid obstructing the deck slot 127.

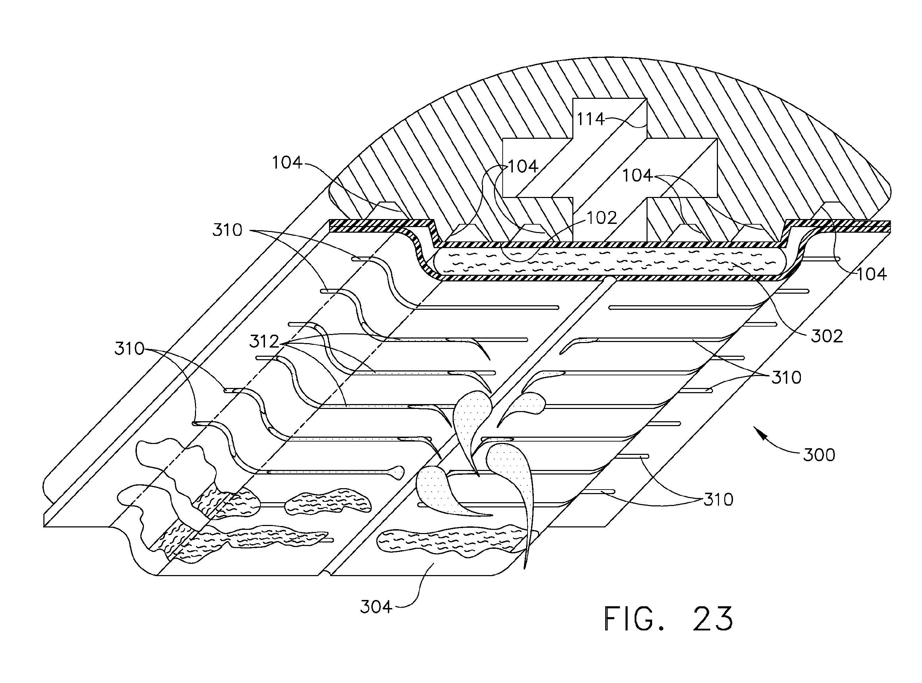

FIG. 23 illustrates another tissue thickness compensator embodiment 300 that is removably attachable to an anvil 100. The tissue thickness compensator 300 comprises a compressible body or foam member 302 that may comprise any of the various foam compositions/configurations describe above. The foam member 302 is encased or sealed or protected from fluids by a film 304. The film 304 may comprise a film fabricated from 65/35PCL/PGA. However, the film may be fabricated from any of the absorbable polymers or their copolymers described above. The tissue thickness compensator 300 may be removably attached to the staple-forming surface 102 of the anvil 100 by employing any of the protrusion arrangements disclosed herein. In this embodiment, the film 304 is substantially water soluble and may have a plurality of areas 310 of nano-wicking features 312 formed therein. The nano-wicking features may serve to encourage hydrophilic wicking which may assist in the melting away of the film 304. Alternatively, the tissue thickness compensator 300 may be attached to the staple cartridge 120 by any of the protrusion arrangements disclosed herein.

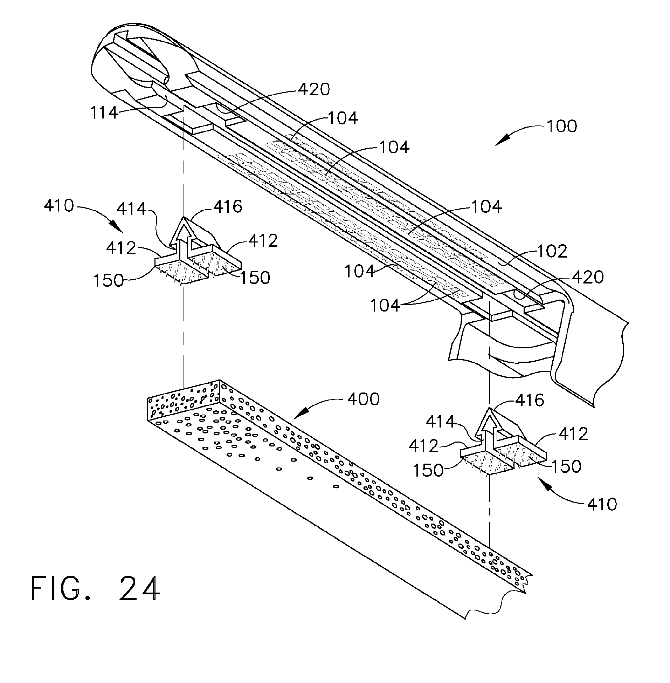

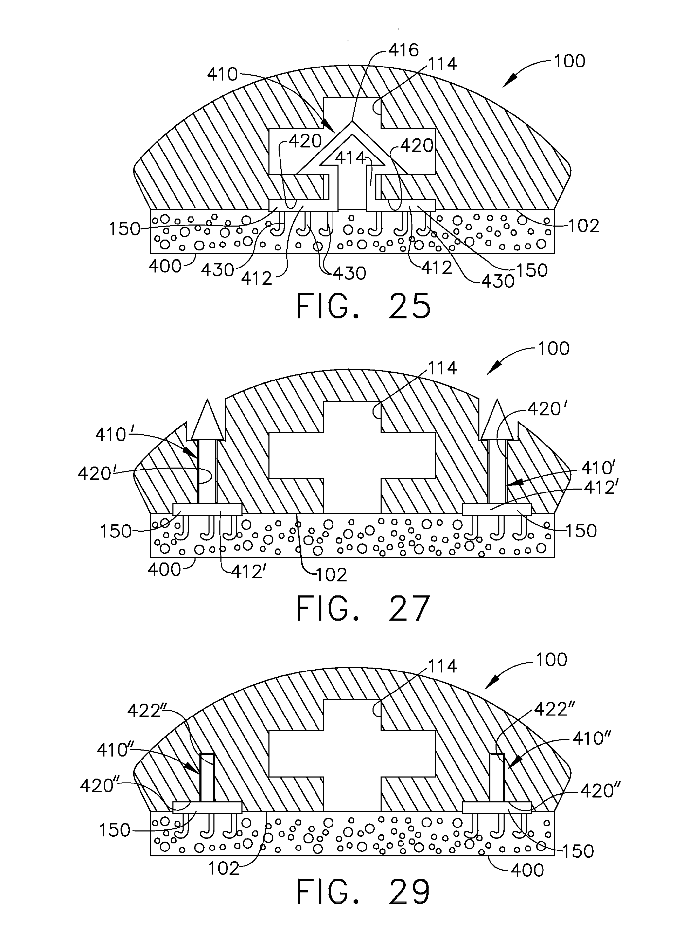

FIGS. 24 and 25 illustrate an alternative embodiment wherein two areas 150 of protrusions are formed on corresponding attachment carriers 410 that are configured to be snapped into corresponding attachment cavities 420 formed in the anvil 100. The attachment carriers 410 depicted in FIGS. 24 and 25 include an attachment deck 412 that has an attachment stem 414 protruding therefrom. The stem 414 terminates in a pointed bayonet-type tip 416 that is configured to snappingly engage the corresponding attachment snap cavity 420. The various forms of protrusions disclosed herein may be integrally formed into the deck 412 of the attachment carrier 410 or they may be attached to the deck 412 by an appropriate adhesive. In FIG. 25, the protrusions 430 are hook-shaped and are well-suited for retainingly hooking a tissue compression member 400 of the various types and compositions described above to the anvil 100. For example, the tissue compression member 400 may comprise a foam or fibrous oxygen regenerated cellulose (ORC) material. The attachment carriers are not implantable and remain with the anvil 100 for reuse. The attachment features 410 are so located such that the protrusion areas 150 are located on each side of the slot 114 in the anvil 100 and such that they do not obstruct or impede any of the staple forming pockets 104. Once the end effector 12 has been fired and the tissue thickness compensator 400 has been cut and stapled to the target tissue, the attachment carriers 410 release the tissue thickness compensator 400 while the carriers 410 remain attached to the anvil 100. Alternatively, the carriers 410 may be attached to the staple cartridge 120 in a similar manner instead of being fastened to the anvil 100.

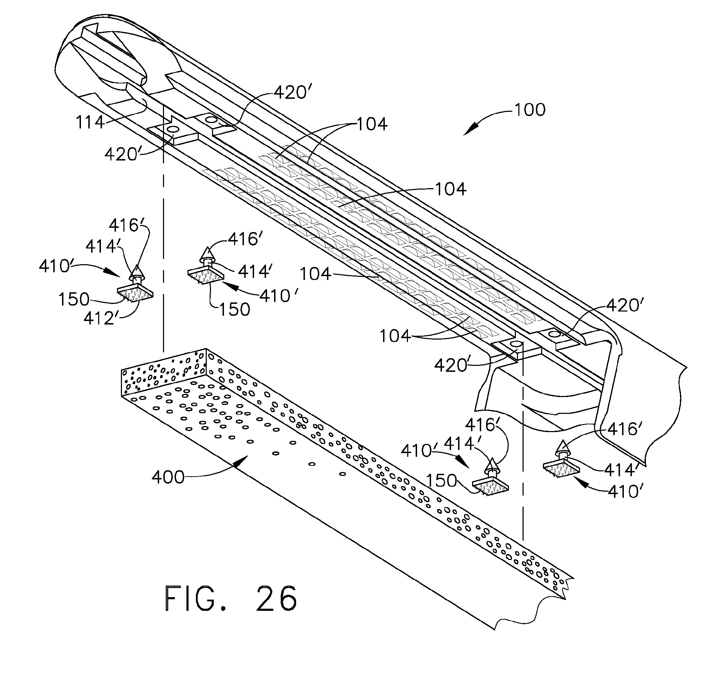

FIGS. 26 and 27 illustrate an alternative embodiment wherein two areas 150 of protrusions are formed on corresponding attachment carriers 410' that are configured to be snapped into corresponding attachment cavities 420' formed in the anvil 100. The attachment carriers 410' depicted in FIGS. 26 and 27 include an attachment deck 412' that has an attachment stem 414' protruding therefrom. The stem 414' terminates in a pointed tip 416' that is configured to snappingly engage the corresponding attachment snap cavity 420'. The various forms of protrusions disclosed herein may be integrally formed into the deck 412' of the attachment carrier 410' or they may be attached to the deck 412' by an appropriate adhesive. In FIG. 27, the protrusions 430 are hook-shaped and are well-suited for retainingly hooking a tissue compression member 400 of the various types and compositions described above to the anvil 100. For example, the tissue compression member 400 may comprise a foam or fibrous oxygen regenerated cellulose (ORC) material. The attachment carriers are not implantable and remain with the anvil 100 for reuse. The attachment features 410' are so located such that the protrusion areas 150 are located on each side of the slot 114 in the anvil 100 and such that they do not obstruct or impede any of the staple forming pockets 104. Once the end effector 12 has been fired and the tissue thickness compensator 400 has been cut and stapled to the target tissue, the attachment carriers 410 release the tissue thickness compensator 400 while the carriers 410' remain attached to the anvil 100. Alternatively, the carriers 410' may be attached to the staple cartridge 120 in a similar manner instead of being fastened to the anvil 100.

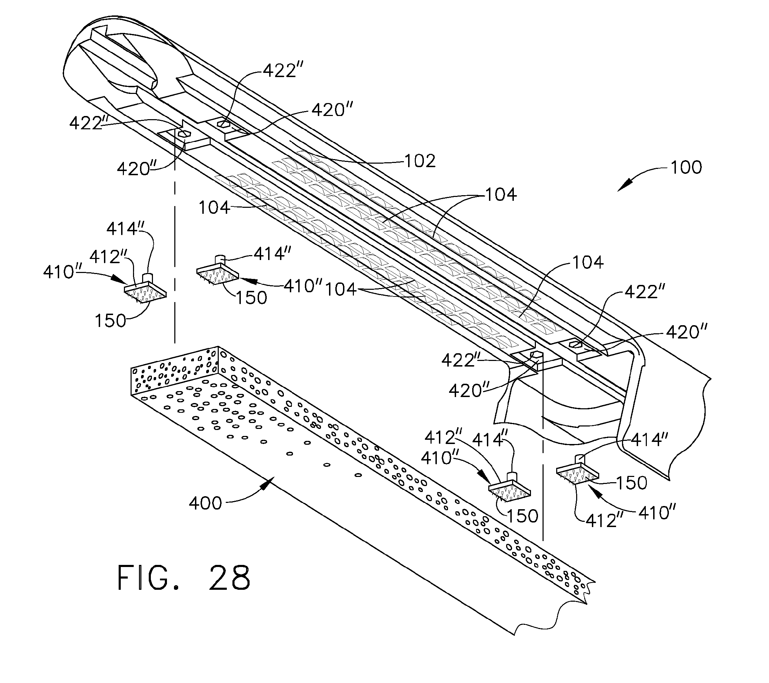

FIGS. 28 and 29 illustrate an alternative embodiment wherein two areas 150 of protrusions are formed on corresponding attachment carriers 410" that are configured to be snapped into corresponding attachment cavities 420" formed in the anvil 100. The attachment carriers 410" depicted in FIGS. 28 and 29 include an attachment deck 412" that has an attachment stem 414" protruding therefrom. As illustrated, the stem 414" has a substantially circular cross-sectional shape and is configured to be retainingly inserted into a corresponding hexagonally-shaped hole 422" in the corresponding attachment snap cavity 420". Alternatively, the attachment stem 414" has a substantially hexagonal cross-sectional shape and the holes 422" are round. The various forms of protrusions disclosed herein may be integrally formed into the deck 412" of the attachment carrier 410" or they may be attached to the deck 412" by an appropriate adhesive. In FIG. 29, the protrusions 430 are hook-shaped and are well-suited for retainingly hooking a tissue compression member 400 of the various types and compositions described above to the anvil 100. For example, the tissue compression member 400 may comprise a foam or fibrous oxygen regenerated cellulose (ORC) material. The attachment carriers are not implantable and remain with the anvil 100 for reuse. The attachment features 410" are so located such that the protrusion areas 150 are located on each side of the slot 114 in the anvil 100 and such that they do not obstruct or impede any of the staple forming pockets 104. Once the end effector 12 has been fired and the tissue thickness compensator 400 has been cut and stapled to the target tissue, the attachment carriers 410 "release the tissue thickness compensator 400 while the carriers 410" remain attached to the anvil 100. Alternatively, the carriers 410" may be attached to the staple cartridge 120 in a similar manner instead of being fastened to the anvil 100.

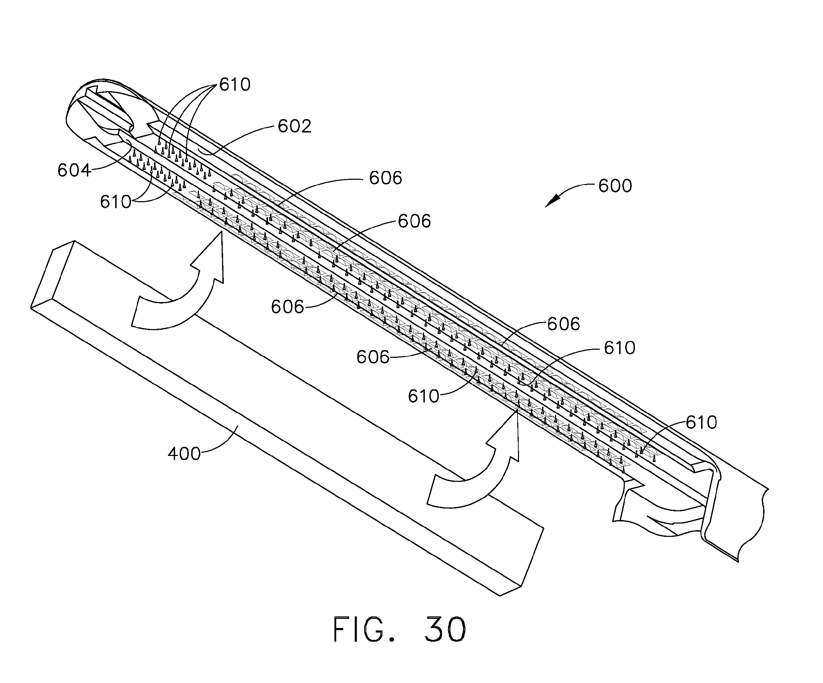

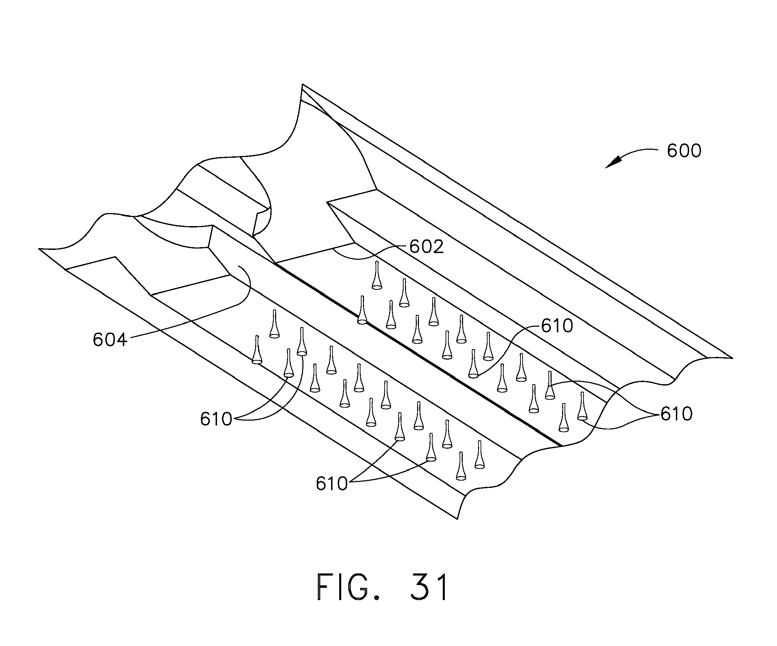

FIGS. 30 and 31 illustrate an alternative anvil 600 that is similar in construction to anvil 100 described above. This embodiment, however, employs a plurality of microfibers 610 formed on the undersurface 602 thereon. For example, the microfibers are formed from a plastic or polymer material and may be attached to underside 602 by an appropriate adhesive and in another arrangement, the tape has microfibers 610 formed on both sides of the tape. The microfibers may have an approximate length of 0.05 to 0.1 inch, and at least 50 microfibers are employed. For example, tape constructions known as "gecko tape" that has the microfibers 610 formed thereon may be attached to the under surface 602 of the anvil 600 in areas on each slide of the longitudinal slot 604 such that they do not interfere with the staple-forming pockets 606 as shown in FIG. 30. The plurality of microfibers 610 serve to removably affix the tissue thickness compensator 400 to the underside 602 of the anvil 600. The microfibers 610 may not attach to the tissue thickness compensator 400 by necessarily being pressed into the surface of the tissue thickness compensator 400, but instead may require a sliding motion parallel to the surface of the tissue thickness compensator 400 for the fibers 610 to bend and attach. Once the end effector has been fired and the tissue thickness compensator 400 has been cut and stapled to the target tissue, the microfibers 610 release from the tissue thickness compensator 400 when the anvil 600 is removed from the stapled tissue. Alternatively, the microfibers may protrude from the deck surface 144 of the staple cartridge 120.

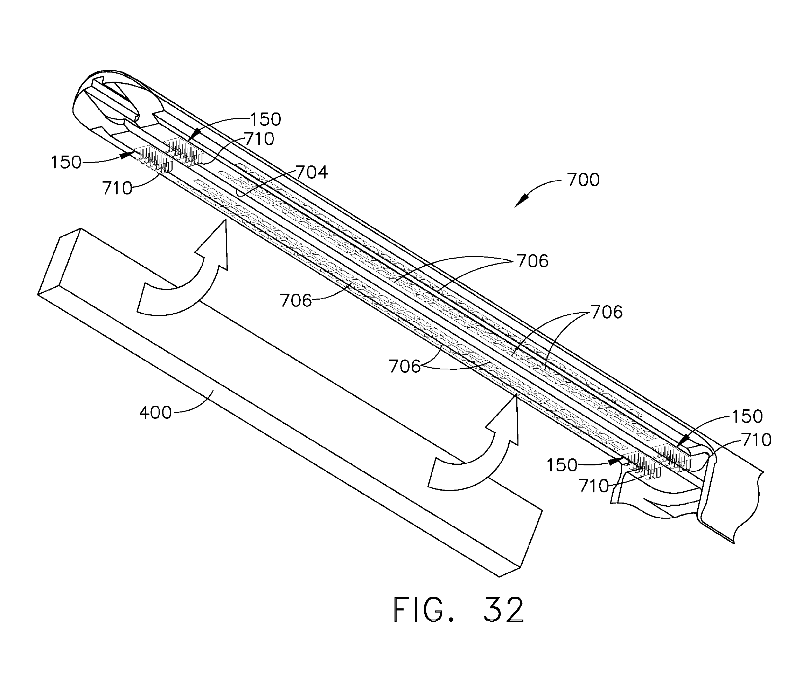

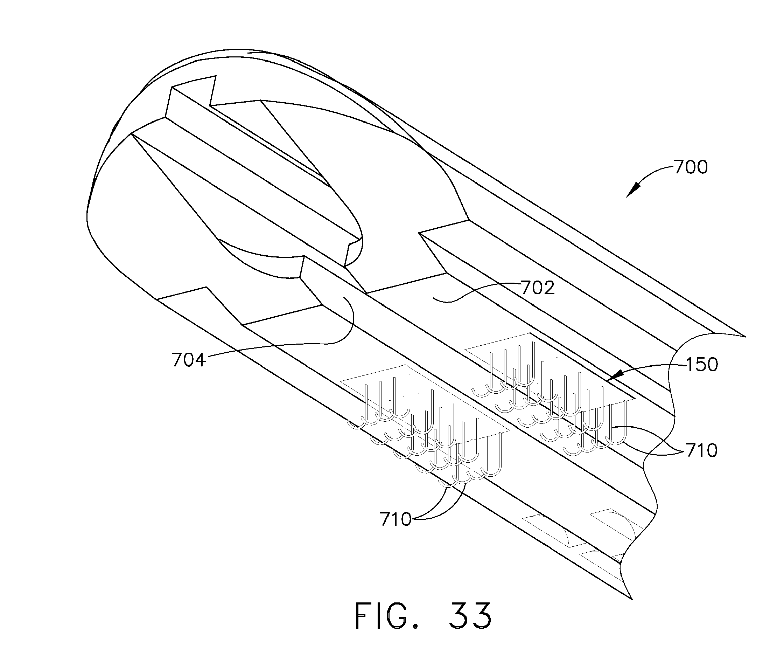

FIGS. 32 and 33 illustrate an alternative anvil 700 that is similar in construction to anvil 100 described above. This embodiment, however, employs at least one area 150 of hook shaped protrusions or fibers 710 that are configured to releasably engage the tissue thickness compensator 400. For example, four areas 150 are located as shown in FIG. 32. As can be seen in that Figure, the protrusions 710 may be formed on a tape arrangement that may be stuck to the underside 702 of the anvil 700. Alternatively, the area(s) 150 are integrally formed in the underside 702 of the anvil 200. The areas 150 may be located on each side of the longitudinal slot 704 such that they do not interfere with the staple-forming pockets 706. The fibers 710 may have an approximate length of 0.05 to 0.1, and each area 150 may have a fiber density of 150-700. Once the end effector has been fired and the tissue thickness compensator 400 has been cut and stapled to the target tissue, the fibers 710 release from the tissue thickness compensator 400 when the anvil 700 is removed from the stapled tissue. Alternatively, the area(s) 150 are provided on the deck 144 of the staple cartridge 120.

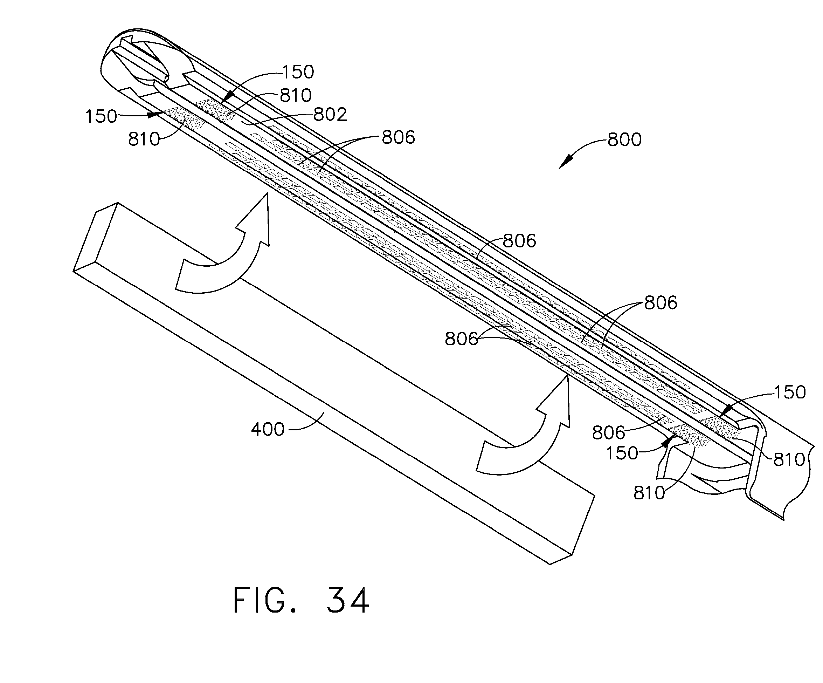

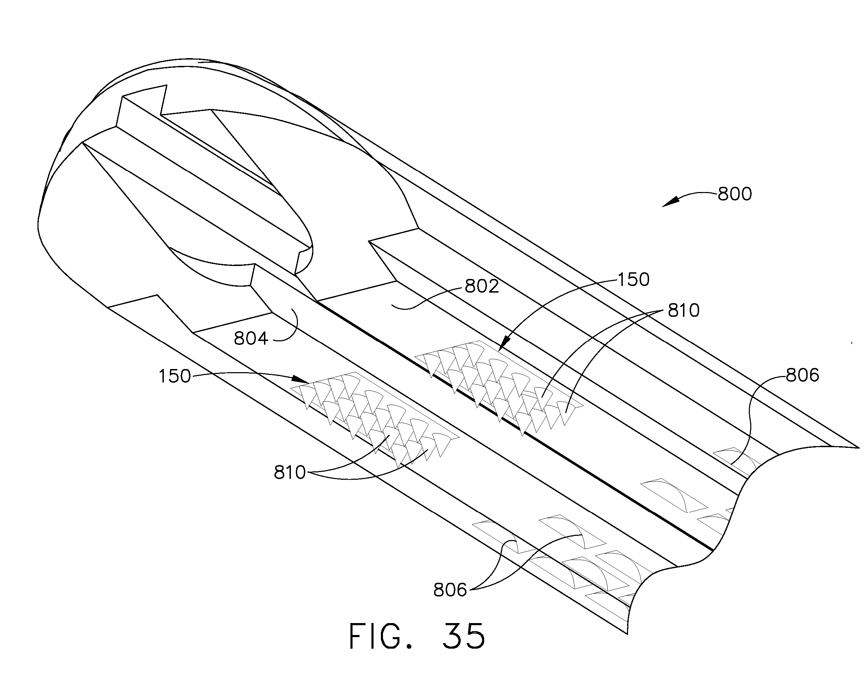

FIGS. 34 and 35 illustrate an alternative anvil 800 that is similar in construction to anvil 100 described above. This embodiment, however, employs at least one area 150 of protrusions 810 that have pointed tips 812 that are configured to pierce into the tissue thickness compensator 400 and releasably attach the tissue thickness compensator 400 to the underside 802 of the anvil 800. The protrusions 810 have a substantial pyramidal shape. Alternatively, the protrusions have an elongated body that has a relatively pointed end to pierce into the tissue thickness compensator 400. For example, four areas 150 are located as shown in FIG. 34. As can be seen in that Figure, the protrusions 810 may be formed on a tape arrangement that may be stuck to the underside 802 of the anvil 800. Alternatively, the protrusions are integrally formed on the underside 802 of the anvil 800. The areas 150 may be located on each slide of the longitudinal slot 804 such that they do not interfere with the staple-forming pockets 806. Once the end effector has been fired and the tissue thickness compensator 400 has been cut and stapled to the target tissue, the protrusions 810 release from the tissue thickness compensator 400 when the anvil 800 is removed from the stapled tissue. Alternatively, the area(s) 150 are provided on the deck 144 of the staple cartridge 120.

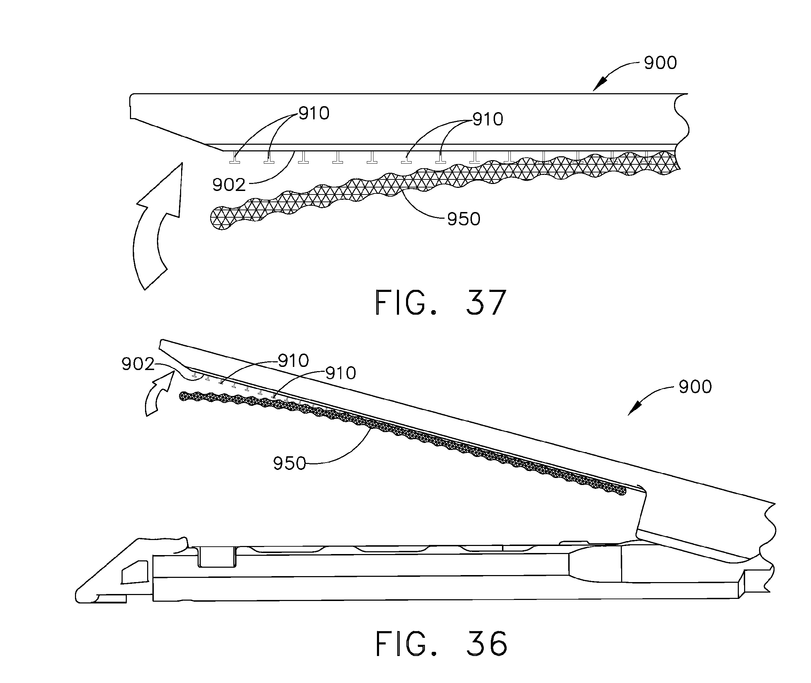

FIGS. 36 and 37 illustrate an alternative anvil 900 that is similar in construction to anvil 100 described above. This embodiment, however, employs a plurality of protrusions 910 that have a substantial T-shape as shown. The protrusions 910 may be formed on a tape arrangement that may be stuck to the underside 902 of the anvil 900. Alternatively, the protrusions may be integrally formed in the underside 902 of the anvil 900. The protrusions are configured to releasably engage a tissue thickness compensator 950 that comprises woven fibrous oxygen regenerated cellulose (ORC) or similar material. Once the end effector has been fired and the tissue thickness compensator 950 has been cut and stapled to the target tissue, the protrusions 910 release from the tissue thickness compensator 950 when the anvil 900 is removed from the stapled tissue. Alternatively, the area(s) 150 are provided on the deck 144 of the staple cartridge 120.

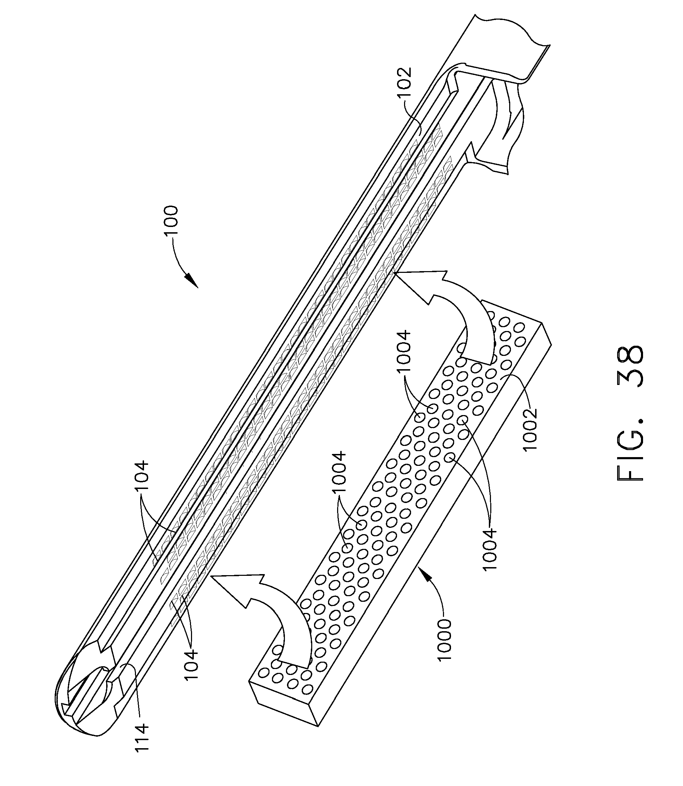

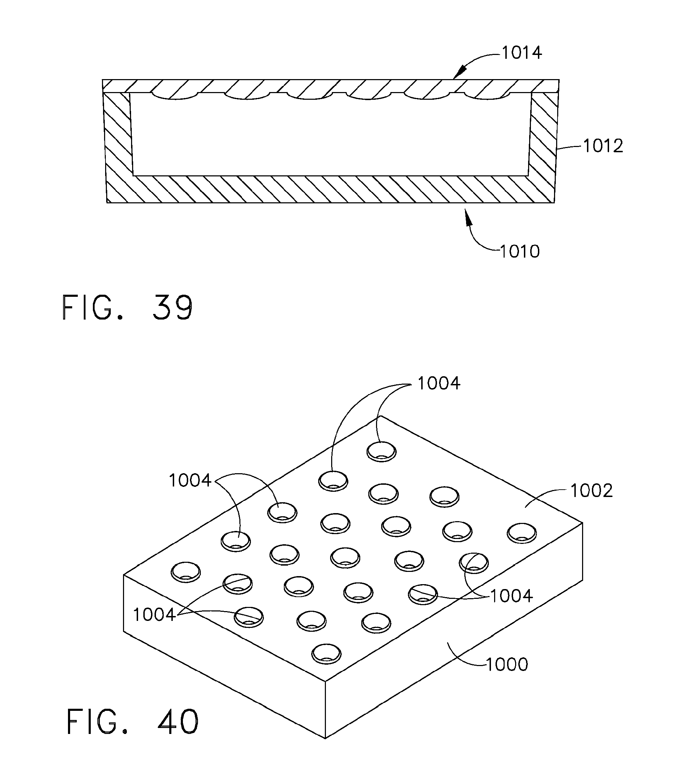

FIGS. 38-40 illustrate an alternative tissue thickness compensator 1000 that is constructed to be removably attachable to the underside 102 of an anvil 100 or to the deck surface 144 of a staple cartridge 120. In this embodiment, the tissue thickness compensator may be fabricated from the various absorbable polymer foams described above. Solid elements of a dissimilar polymer are added that will not be dissolved by the solvent that is core to the foam molding. For instance PLA/PCL can be suspended in chlorophyll which will not dissolve any PGA-based materials. The cavities are made from non-dissolvable materials. In at least one arrangement, the mold 1010 has a body portion 1012 that defines a cavity into which the material is introduced. The mold 1010 further has a lid 1014 that is configured to form an array of suction cup formations 1004 in an upper surface 1002 of the tissue thickness compensator 1000. To removably attach the tissue thickness compensator 1000 to the anvil 100, the upper surface 1002 is pressed into engagement with the underside 102 of the anvil 100. The suction formations 1004 serve to removably adhere the tissue thickness compensator 1000 to the anvil 100. Alternatively, the suction formations 1004 are configured to removably adhere the tissue thickness compensator 1000 to the deck 142 of the surgical staple cartridge 120.

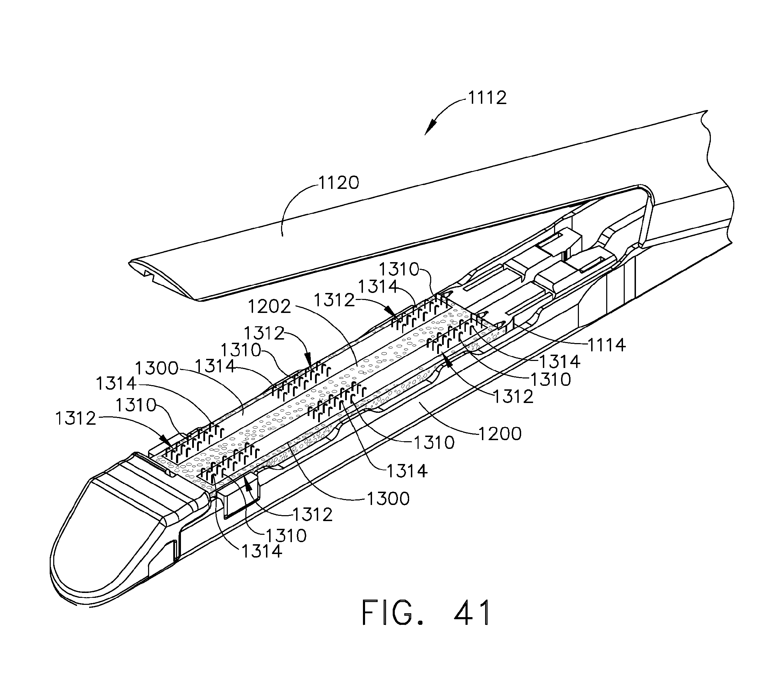

FIG. 41 illustrates an end effector 1112 that has a cartridge 1114, a tissue thickness compensator 1200 supported on the cartridge 1114, and an anvil 1120. U.S. Patent Application Serial No. 13/097,891, entitled "Tissue Thickness Compensator For a Surgical Stapler Comprising an Adjustable Anvil", which has been previously herein incorporated by reference in its entirety discloses examples of such end effector arrangements. FIG. 28 employs a pair of tissue engagement strips 1300 that are attached to the upper surface 1202 of the tissue thickness compensator 1200. The tissue engagement strips 1300 comprise adhesive strips that have tissue engaging protrusions 1310 protruding therefrom. The strips 1300 are located on each side of the slot (not shown) in the staple cartridge 1114 that accommodates the tissue cutting member (not shown). The strips 1300 may have the protrusions 1310 evenly distributed throughout their entire length or they may be arranged in discrete zones or areas 1312. For example, In FIG. 28, the protrusions 1310 are arranged in three areas 1312 on each strip 1300. The protrusions 1310 may comprise any of the protrusion configurations disclosed herein. As illustrated, for example, each protrusion 1310 has a hook 1314 formed on its end to engage the tissue that gets clamped between the anvil 1120 and the tissue thickness compensator 1200. The tissue engagement strips 1300 may comprise, for example, hook tape strips sold under the trademark "Velcro". The strips 1300 may be attached to the tissue thickness compensator 1200 by adhesive, stitching or other suitable fastening arrangements. On compression, the protrusions 1310 engage the clamped tissue to improve the traction on the tissue.

Various embodiments disclosed herein and their respective equivalent structures are particularly well-suited for attaching fibrous or foam tissue thickness compensator arrangements to portions of a surgical stapling instrument. For example, various protrusion arrangements disclosed herein are configured to "mechanically retainingly engage" the structure of the tissue thickness compensator to removably attach the tissue thickness compensator to a portion of the surgical stapling instrument. As used herein, the term "mechanically retainingly engage" is meant to encompass forms of retaining engagement between corresponding protrusions and/or between the protrusions and the tissue thickness compensator structure for fastening the tissue thickness compensator to a portion of the surgical stapling instrument without the use of chemical adhesives to complete the bond. Other protrusion arrangements disclosed herein are well-suited for retainingly engaging tissue thickness compensators that are fabricated from foam material. These arrangements may be distinguished from those surgical stapler arrangements where a substantially smooth, Mylar-like buttress material is used and wherein a chemical adhesive is employed for establishing the bond between the buttress material and the stapling instrument structure. In addition, while areas of attachment protrusions have been herein disclosed with respect to certain embodiments, in other cases, only one attachment protrusion may be employed that is configured and shaped to mechanically interface with the structure of the tissue thickness compensator to removably adhere the tissue thickness compensator to a portion of the surgical stapling instrument. For example, the attachment protrusion may comprise a protrusion terminating in a hook-like structure that is well suited to hookingly engage a fibrous or woven portion of the tissue thickness compensator.

In addition, it will be understood that the various embodiments disclosed herein may be effectively employed with a variety of different surgical stapler arrangements. For example, in addition to the various surgical stapling devices depicted in the Figures, various embodiments may be effectively employed with open staplers, linear staplers, circular staplers, etc. Such staplers may be manually controlled, motor controlled and/or robotically controlled. In addition, while various embodiments have been described herein connection with attaching tissue thickness compensating materials to the anvil or surgical stapling cartridge of a surgical stapling device, various embodiments may be employed to attach tissue thickness compensators to other portion(s) of the surgical stapler.

Further to the above, a tissue thickness compensator can be comprised of a biocompatible material. The biocompatible material, such as, a foam, may comprise tackifiers, surfactants, fillers, cross-linkers, pigments, dyes, antioxidants and other stabilizers and/or combinations thereof to provide desired properties to the material. A biocompatible foam may comprise a surfactant. The surfactant may be applied to the surface of the material and/or dispersed within the material. Without wishing to be bound to any particular theory, the surfactant applied to the biocompatible material may reduce the surface tension of the fluids contacting the material. For example, the surfactant may reduce the surface tension of water contacting the material to accelerate the penetration of water into the material. The water may act as a catalyst. The surfactant may increase the hydrophilicity of the material.

The surfactant may comprise an anionic surfactant, a cationic surfactant, and/or a nonionic surfactant. Examples surfactants include, but are not limited to polyacrylic acid, methalose, methyl cellulose, ethyl cellulose, propyl cellulose, hydroxy ethyl cellulose, carboxy methyl cellulose, polyoxyethylene cetyl ether, polyoxyethylene lauryl ether, polyoxyethylene octyl ether, polyoxyethylene octylphenyl ether, polyoxyethylene oleyl ether, polyoxyethylene sorbitan monolaurate, polyoxyethylene stearyl ether, polyoxyethylene nonylphenyl ether, dialkylphenoxy poly(ethyleneoxy) ethanol, and polyoxamers, and combinations thereof. The surfactant may comprise a copolymer of polyethylene glycol and polypropylene glycol. The surfactant may comprise a phospholipid surfactant. The phospholipid surfactant may provide antibacterial stabilizing properties and/or disperse other materials in the biocompatible material. The tissue thickness compensator may comprise at least one medicament. The tissue thickness compensator may comprise one or more of the natural materials, non-synthetic materials, and/or synthetic materials described herein. The tissue thickness compensator may comprise a biocompatible foam comprising gelatin, collagen, hyaluronic acid, oxidized regenerated cellulose, polyglycolic acid, polycaprolactone, polyactic acid, polydioxanone, polyhydroxyalkanoate, poliglecaprone, and combinations thereof The tissue thickness compensator may comprise a film comprising the at least one medicament. The tissue thickness compensator may comprise a biodegradable film comprising the at least one medicament. The medicament may comprise a liquid, gel, and/or powder. The medicaments may comprise anticancer agents, such as, for example, cisplatin, mitomycin, and/or adriamycin.

The tissue thickness compensator may comprise a biodegradable material to provide controlled elution of the at least one medicament as the biodegradable material degrades. The biodegradable material may degrade may decompose, or loses structural integrity, when the biodegradable material contacts an activator, such as, for example an activator fluid. The activator fluid may comprise saline or any other electrolyte solution, for example. The biodegradable material may contact the activator fluid by conventional techniques, including, but not limited to spraying, dipping, and/or brushing. In use, for example, a surgeon may dip an end effector and/or a staple cartridge comprising the tissue thickness compensator comprising the at least one medicament into an activator fluid comprising a salt solution, such as sodium chloride, calcium chloride, and/or potassium chloride. The tissue thickness compensator may release the medicament as the tissue thickness compensator degrades. The elution of the medicament from the tissue thickness compensator may be characterized by a rapid initial elution rate and a slower sustained elution rate.

According to the invention, a tissue thickness compensator, for example, can be comprised of a biocompatible material which may comprise an oxidizing agent. The oxidizing agent may an organic peroxide and/or an inorganic peroxide. Examples of oxidizing agents may include, but are not limited to, hydrogen peroxide, urea peroxide, calcium peroxide, and magnesium peroxide, and sodium percarbonate. The oxidizing agent may comprise peroxygen-based oxidizing agents and hypohalite-based oxidizing agents, such as, for example, hydrogen peroxide, hypochlorous acid, hypochlorites, hypocodites, and percarbonates. The oxidizing agent may comprise alkali metal chlorites, hypochlorites and perborates, such as, for example, sodium chlorite, sodium hypochlorite and sodium perborate. The oxidizing agent may comprise vanadate. The oxidizing agent may comprise ascorbic acid. The oxidizing agent may comprise an active oxygen generator. According to the invention, a tissue scaffold may comprise the biocompatible material comprising an oxidizing agent.

The biocompatible material may comprise a liquid, gel, and/or powder. The oxidizing agent may comprise microparticles and/or nanoparticles, for example. For example, the oxidizing agent may be milled into microparticles and/or nanoparticles. The oxidizing agent may be incorporated into the biocompatible material by suspending the oxidizing agent in a polymer solution. The oxidizing agent may be incorporated into the biocompatible material during the lyophylization process. After lyophylization, the oxidizing agent may be attached to the cell walls of the biocompatible material to interact with the tissue upon contact. The oxidizing agent may not be chemically bonded to the biocompatible material. A percarbonate dry power may be embedded within a biocompatible foam to provide a prolonged biological effect by the slow release of oxygen. A percarbonate dry power may be embedded within a polymeric fiber in a non-woven structure to provide a prolonged biological effect by the slow release of oxygen. The biocompatible material may comprise an oxidizing agent and a medicament, such as, for example, doxycycline and ascorbic acid.

The biocompatible material may comprise a rapid release oxidizing agent and/or a slower sustained release oxidizing agent. The elution of the oxidizing agent from the biocompatible material may be characterized by a rapid initial elution rate and a slower sustained elution rate. The oxidizing agent may generate oxygen when the oxidizing agent contacts bodily fluid, such as, for example, water. Examples of bodily fluids may include, but are not limited to, blood, plasma, peritoneal fluid, cerebral spinal fluid, urine, lymph fluid, synovial fluid, vitreous fluid, saliva, gastrointestinal luminal contents, and/or bile. Without wishing to be bound to any particular theory, the oxidizing agent may reduce cell death, enhance tissue viability and/or maintain the mechanical strength of the tissue to tissue that may be damaged during cutting and/or stapling.
The biocompatible material may comprise at least one microparticle and/or nanoparticle. The biocompatible material may comprise one or more of the natural materials, non-synthetic materials, and synthetic materials described herein. The biocompatible material may comprise particles having a mean diameter of about 10 nm to about 100 nm and/or about 10 µm to about 100 µm, such as, for example, 45-50 nm and/or 45-50 µm. The biocompatible material may comprise biocompatible foam comprising at least one microparticle and/or nanoparticle embedded therein. The microparticle and/or nanoparticle may not be chemically bonded to the biocompatible material. The microparticle and/or nanoparticle may provide controlled release of the medicament. The microparticle and/or nanoparticle may comprise at least one medicament. The microparticle and/or nanoparticle may comprise a hemostatic agent, an anti-microbial agent, and/or an oxidizing agent, for example. The tissue thickness compensator may comprise a biocompatible foam comprising an hemostatic agent comprising oxidized regenerated cellulose, an anti-microbial agent comprising doxycline and/or Gentamicin, and/or an oxidizing agent comprising a percarbant. The microparticle and/or nanoparticle may provide controlled release of the medicament up to three days, for example.

The microparticle and/or nanoparticle may be embedded in the biocompatible material during a manufacturing process. For example, a biocompatible polymer, such as, for example, a PGA/PCL, may contact a solvent, such as, for example, dioxane to form a mixture. The biocompatible polymer may be ground to form particles. Dry particles, with or without ORC particles, may be contacted with the mixture to form a suspension. The suspension may be lyophilized to form a biocompatible foam comprising PGA/PCL having dry particles and/or ORC particles embedded therein.

The tissue thickness compensators or layers disclosed herein can be comprised of an absorbable polymer, for example. A tissue thickness compensator can be comprised of foam, film, fibrous woven, fibrous non-woven PGA, PGA/PCL (Poly( glycolic acid-co-caprolactone)), PLA/PCL (Poly(lactic acid-co- polycaprolactone)), PLLA/PCL, PGA/TMC (Poly(glycolic acid-co-trimethylene carbonate)), PDS, PEPBO or other absorbable polyurethane, polyester, polycarbonate, Polyorthoesters, Polyanhydrides, Polyesteramides, and/or Polyoxaesters, for example. According to the invention, a tissue thickness compensator can be comprised of PGA/PLA (Poly(glycolic acid-co-lactic acid)) and/or PDS/PLA (Poly(p-dioxanone-co-lactic acid)), for example. According to the invention, a tissue thickness compensator can be comprised of an organic material, for example. A tissue thickness compensator can be comprised of Carboxymethyl Cellulose, Sodium Alginate, Cross-linked Hyaluronic Acid, and/or Oxidized regenerated cellulose, for example. According to the invention, a tissue thickness compensator can comprise a durometer in the 3-7 Shore A (30-50 Shore OO) ranges with a maximum stiffness of 15 Shore A (65 Shore OO), for example. A tissue thickness compensator can undergo 40% compression under 3 lbf load, 60% compression under 6 lbf load, and/or 80% compression under 20 lbf load, for example. One or more gasses, such as air, nitrogen, carbon dioxide, and/or oxygen, for example, can be bubbled through and/or contained within the tissue thickness compensator. A tissue thickness compensator can comprise beads therein which comprise between approximately 50% and approximately 75% of the material stiffness comprising the tissue thickness compensator.

According to the invention, a tissue thickness compensator can comprise hyaluronic acid, nutrients, fibrin, thrombin, platelet rich plasma, Sulfasalazine (Azulfidine® - 5ASA+Sulfapyridine diazo bond))- prodrug - colonic bacterial (Azoreductase), Mesalamine (5ASA with different prodrug configurations for delayed release), Asacol® (5ASA + Eudragit-S coated - pH > 7 (coating dissolution)), Pentasa® (5ASA + ethylcellulose coated- time/pH dependent slow release), Mesasal® (5ASA + Eudragit-L coated-pH > 6), Olsalazine (5ASA + 5ASA - colonic bacterial (Azoreductase)), Balsalazide (5ASA + 4Aminobenzoyl-B-alanine) - colonic bacterial (Azoreductase)), Granulated mesalamine, Lialda (delay and SR formulation of mesalamine), HMPL-004 (herbal mixture that may inhibit TNF-alpha, interleukin-1 beta, and nuclear-kappa B activation), CCX282-B (oral chemokine receptor antagonist that interferes with trafficking of T lymphocytes into the intestinal mucosa), Rifaximin (nonabsorbable broad-spectrum antibiotic ), Infliximab, murine chymieric (monoclonal antibody directed against TNF-alpha-approved for reducing signs/symptoms and maintaining clinical remission in adult/pediatric patients with moderate/severe luminal and fistulizing Crohn's disease who have had inadequate response to conventional therapy), Adalimumab, Total Human IgG1 (anti-TNF-alpha monoclonal antibody - approved for reducing signs/symptoms of Crohn's disease, and for the induction and maintenance of clinical remission in adult patients with moderate/severe active Crohn's disease with inadequate response to conventional therapies, or who become intolerant to Infliximab), Certolizumab pegoll, humanized anti-TNF FAB' (monoclonal antibody fragment linked to polyethylene glycol - approved for reducing signs/symptoms of Crohn's disease and for the induction and maintenance of response in adult patients w/ moderate/severe disease with inadequate response to conventional therapies), Natalizumab, First non-TNF-alpha inhibitor (biologic compound approved for Crohn's disease), Humanized monoclonal IgG4 antibody (directed against alpha-4 integrin - FDA approved for inducing and maintaining clinical response and remission in patients with moderate/severe disease with evidence of inflammation and who have had inadequate response to or are unable to tolerate conventional Crohn's therapies and inhibitors of TNF-alpha), concomitant Immunomodulators potentially given with Infliximab, Azathioprine 6-Mercaptopurine (purine synthesis inhibitor - prodrug), Methotrexate (binds dihydrofolate reductase (DHFR) enzyme that participates in tetrahydrofolate synthesis, inhibits all purine synthesis), Allopurinol and Thioprine therapy, PPI, H2 for acid suppression to protect the healing line, C-Diff - Flagyl, Vancomycin (fecal translocation treatment; probiotics; repopulation of normal endoluminal flora), and/or Rifaximin (treatment of bacterial overgrowth (notably hepatic encephalopathy); not absorbed in GI tract with action on intraluminal bacteria), for example.

As described herein, a tissue thickness compensator can compensate for variations in the thickness of tissue that is captured within the staples ejected from a staple cartridge and/or contained within a staple line, for example. Stated another way, certain staples within a staple line can capture thick portions of the tissue while other staples within the staple line can capture thin portions of the tissue. In such circumstances, the tissue thickness compensator can assume different heights or thicknesses within the staples and apply a compressive force to the tissue captured within the staples regardless of whether the captured tissue is thick or thin. According to the invention, a tissue thickness compensator can compensate for variations in the hardness of the tissue. For instance, certain staples within a staple line can capture highly compressible portions of the tissue while other staples within the staple line can capture portions of the tissue which are less compressible. In such circumstances, the tissue thickness compensator can be configured to assume a smaller height within the staples that have captured tissue having a lower compressibility, or higher hardness, and, correspondingly, a larger height within the staples that have captured tissue having a higher compressibility, or lower hardness, for example. In any event, a tissue thickness compensator, regardless of whether it compensates for variations in tissue thickness and/or variations in tissue hardness, for example, can be referred to as a 'tissue compensator' and/or as a 'compensator', for example.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

While this invention has been described as having exemplary designs, the present invention may be further modified within the spirit and scope of the disclosure. This application is therefore intended to cover any variations, uses, or adaptations of the invention using its general principles. Further, this application is intended to cover such departures from the present disclosure as come within known or customary practice in the art to which this invention pertains.

## Claims

1. A surgical stapling instrument comprising:
a first jaw supporting a plurality of surgical staples therein operably responsive to an application of a firing motion thereto;
a second jaw movably supported relative to the first jaw such that a portion of the second jaw is movable into confronting relationship relative to the first jaw upon application of a closing motion to the second jaw;
a tissue thickness compensator configured to be captured within the surgical staples and assume different compressed heights within different surgical staples upon application of the firing motion to the surgical staples; and
at least one attachment protrusion on one of the first and second jaws for removably mechanically affixing a corresponding portion of the tissue thickness compensator thereto.

2. The surgical stapling instrument of claim 1 wherein the tissue thickness compensator comprises a fibrous body structure and wherein the at least one attachment protrusion comprises a plurality of attachment protrusions configured to removably retainingly engage the fibrous body structure.

3. The surgical stapling instrument of claim 1 or claim 2 wherein the tissue thickness compensator comprises a compressible body encapsulated by a film and wherein the at least one attachment protrusion is configured to removably retainingly engage at least one other corresponding other attachment protrusion on the film.

4. The surgical stapling instrument of any preceding claim wherein the at least one attachment protrusion comprises at least one area of:
attachment protrusions having a pyramidal shape;
attachment protrusions having a hexagonal shape;
attachment protrusions including a protruding body portion and a distal end portion that is not coaxial with the body portion;
attachment columns including a height that is greater than its cross-sectional area;
hook-shaped attachment protrusions;
loop-shaped protrusions;
T-shaped protrusions; and
microfibers.

5. The surgical stapling instrument of claim 3 wherein the film includes at least one area of fluid-wicking members formed thereon.

6. The surgical stapling instrument of any preceding claim wherein the tissue thickness compensator comprises a compressible body fabricated from woven material, or a compressible body fabricated from non-woven material.

7. The surgical stapling instrument of any preceding claim wherein the first jaw comprises a cartridge body defining a deck surface including a plurality of staple cavities through the deck surface and wherein the plurality of surgical staples are positioned within the staple cavities.

8. The surgical stapling instrument of claim 7 wherein the at least one attachment protrusion protrudes from the deck surface.

9. The surgical stapling instrument of claim 7 or claim 8 wherein the at least one attachment protrusion is integrally formed on the deck surface.

10. The surgical stapling instrument of any preceding claim wherein the cartridge body includes a longitudinally extending deck slot formed through the deck surface for receiving a portion of a tissue cutting member therein and wherein the at least one attachment protrusion comprises at least one area of attachment protrusions on one side of the deck slot and at least one other area of attachment protrusions on another side of the deck slot.

11. The surgical stapling instrument of any preceding claim wherein the second jaw comprises an anvil including a staple-forming surface thereon and wherein the at least one attachment protrusion protrudes from the staple forming surface.

12. The surgical stapling instrument of claim 11 wherein the at least one attachment protrusion comprises an area of attachment protrusions formed on a corresponding attachment carrier that is configured to be retainingly coupled to the anvil.

13. The surgical stapling instrument of claim 12 wherein each of the corresponding attachment carriers is configured to snappingly engage the anvil.

14. The surgical stapling instrument of claim 12 or claim 13 wherein the area of attachment protrusions are attached to the corresponding attachment carrier by adhesive, or integrally formed on the corresponding attachment carrier.

15. A surgical stapling instrument, comprising:
a staple cartridge, comprising:
a cartridge body defining a deck surface having a plurality of staple cavities through the deck surface;
a plurality of staples positioned within the staple cavities;
an anvil including a staple-forming surface thereon and being movably supported relative to the staple cartridge to bring the staple forming surface in confronting relationship relative to the deck surface of the cartridge body in response to closing motions applied thereto;
a tissue thickness compensator configured to be captured within the staples and assume different compressed heights within different staples; and
at least one area of attachment protrusions on the deck surface or on the staple forming surface of the anvil for removably attaching the tissue thickness compensator thereto.
